# EUROPEAN PATENT SPECIFICATION

(11) **EP 1 485 154 B1**
(45) Date of publication and mention of the grant of the patent: **07.05.2008**
(21) Application number: 02807120.7
(22) Date of filing: 10.10.2002
(51) Int. Cl.: A61M 15/00

(54) **POWDER INHALATION DEVICE**
INHALATIONSGERÄT FÜR PULVERFÖRMIGE SUBSTANZEN
DISPOSITIF D'INHALATION DE POUDRE

(30) Priority: 20.03.2002 WO PCT/US02/08298
(43) Date of publication of application: 15.12.2004
(73) Proprietor: Alkermes, Inc., Cambridge MA 02139 (US)
(72) Inventor: EDWARDS, David, Boston, MA 02116 (US); ELLWANGER, Colleen, Lexington, MA 02420 (US); FOSHEE, David, L., Apex, NC 27523 (US); DURKIN, Jason, R., Apex, NC 27523 (US); COKER, Timothy, B., Merrimack, NH 03054 (US); STAPLETON, Kevin, Seattle, Washington 98112 (US); DREESEN, Sarah, Medford, MA 02155 (US); DELONG, Mark, Newton, MA 02466 (US); JONES, Andrew, Roslindale, MA 02131 (US); MCMANUS, Ryan, Cambridge, MA 02139 (US); SAUNDERS, Margaret, Millar, Cambridge, MA 02142 (US); SPALLER, Robert, W., Amesbury, MA 01913 (US); ZIEGLER, Andrew, Arlington, MA 02476 (US)
(74) Representative: Lee, Nicholas John
(86) International application number: PCT/US2002/032264
(87) International publication number: WO 2003/080163

(56) References cited:
- EP-A- 0 407 276
- EP-A- 0 506 292
- EP-A- 1 062 962
- US-A- 4 069 819
- US-A- 6 089 228
- US-A- 6 116 237

## Description

### Background of the Invention

### Field of the Invention

The present invention relates generally to facilitating release of powder contained in a receptacle. More specifically, the present invention relates to the administration of medication by an apparatus for facilitating inhalation of powder medicaments.

### Related Art

In the medical field, it is often desirable to administer various forms of medication to patients. Well known methods of introducing medication into the human body include the oral ingestion of capsules and tablets, intravenous injection through hypodermic needles, and numerous others. In one method, certain medications may be inhaled into a patient's respiratory tract and lungs through the nose or mouth. Certain of these medications, such as bronchodilators, corticosteroids, etc., for the treatment of asthma and other respiratory anomalies, may be aimed at the respiratory tract directly, Others are inhaled for purposes of systemic treatment, *i.e.* for treatment of any area of the body through absorption from the respiratory tract through the lung tissue, into the deep lungs, and into the bloodstream. Each of these medications comes in a variety of forms, including fluids, which are commonly administered as an aerosol vapor or mist, as well as solids. Inhalable solids typically take the form of fine, dry powders. Specialized devices, such as inhalers, are provided to assist the patient in directing these fine powder medications into the respiratory tract.

Various types of inhalers are known for the administration of dry powder medicaments. However, each of these inhalers suffers certain drawbacks. For example, U.S. Patent No. 5,787,881 discloses an inhaler that is used with encapsulated dry powder medicaments. However, use of this device requires numerous steps and imposes a number of inconveniences on a user. For example, the medication capsules used with the device have an aperture formed therein prior to insertion into an opening in the inhaler. Therefore, there exists a danger that an amount of medication may be lost prior to or during insertion into the device. After insertion of the capsule, use of the device requires the additional step.that a cover must be closed before the medication may be inhaled.

Inhalation devices configured for use with a capsule containing some type of medicament are shown in U.S. Patent No. 4,069,819 to Valentini et al. ("the '819 patent") and U.S. Patent No. 4,995,385 to Valentini et al. ("the '385 patent"). The inhalation device described in the '385 patent was developed to overcome the drawbacks of the device described in the '819 patent. Particularly, in a large number of cases, the device described in the '819 patent experienced irregular and incomplete emptying of the capsule, thereby resulting in difficulties in properly administering the medicament in the capsule. The inhalation device described in the '385 patent attempts to overcome this deficiency by tapering the nebulization chamber toward the end surface that comprises the discharge holes. Thus, the nebulization chamber of the '385 patent is not cylindrical, but rather frusto-conical in form in an attempt to achieve regular complete emptying of the nebulization chamber.

However, further improvements in the design of inhalation devices are needed to achieve high emitted doses and highly dispersed powders while maintaining low resistance, especially when the inhaler is used with high doses and is operated at low peak inspiratory flow rates (PIFR) and low inhalation volumes. As used herein, "emitted dose" (ED) refers to the percentage of the dose of powder medicament that is emitted from a receptacle in the inhalation device. The dispersal of the powder can be quantified by measuring the volume mean geometric diameter (VMGD) of the emitted powder. As used herein "volume mean geometric diameter" refers to the average geometric diameter of the powder. As used herein, "resistance" refers to the square root of the pressure gradient across the inhaler divided by the peak inspiratory flow rate through the inhaler. As used herein "low peak inspiratory flow rate" refers to a peak inspiratory flow rate of approximately 25 L/min or less. Moreover, improvements are needed to achieve high emitted doses and highly dispersed powders that are consistently reproducible, *i*.*e*., that have a low standard deviation of emitted dose percentage and VMGD, respectively.

Another drawback of the inhalation devices described in the '819 and the '385 patents is the piercing device that is used to puncture the capsule. Such conventional piercing devices are formed from circular stock, with the points created by pinching the stock at an angle, thereby creating a single sharp cutting edge. Drawbacks of such a design are that the point (which must puncture the capsule material) is often rounded, lessening its effectiveness as a piercing device. Moreover, burrs often form on the lower edge, which can stop the piercing device from retracting from the capsule, thereby causing a device failure. The holes formed by such a conventional piercing device are generally round, and do not have the appearance of being cut by a sharp edge. With such a conventional design, the capsule is often crushed, rather than punctured or pierced. If such a conventional piercing device is used with brittle capsule materials such as gelatin, pieces of capsule material of a size that can be inhaled are usually broken off from the capsule. Thus, conventional piercing devices are less than optimal, particularly for brittle capsule material.

Another drawback of conventional inhalation devices is that they have no means for indicating when the powder in the inhaler is ready for inhalation by the user. It is desirable to have a means for indicating to the user that a dose of powder is ready for inhalation. For example, it would be desirable for a patient using a device, for dispensing fluticasone propionate (used to treat asthma) to know when the device is ready for inhalation.

EP-A-1062962 discloses an inhalation device that has a chamber for a capsule containing a powdered medicine. The device is arranged so that the capsule is pierced, releasing the medicine so that it can be inhaled through an inhalation part of the device.

US-A-6116237 discloses an inhalation device that includes an electric motor that powers an impeller. The impeller acts to mix powdered medicine, contained in a cartridge, with air so that it can be inhaled by a user.

There is a need in the art for an improved method and apparatus for inhalation of dry powder medicaments. What is needed is an inhaler that provides for a higher emitted dose that is consistently reproducible with low standard deviation. Such a need is particularly acute for low peak inspiratory flow rates, and for high dosage ranges. There is a further need in the art for an improved means for puncturing the capsule containing the medicament. The present invention, the description of which is fully set forth below, solves the need in the art for such improved methods and apparatus.

### Summary of the Invention

The present invention provides an inhalation device for administering a dose of powder contained in a receptacle, comprising:
a chamber configured to hold the receptacle, said chamber defined by a wall and having a proximal end, a distal end, and an inner surface, said wall defining a plurality of vents, the chamber optionally being cylindrical and/or having a ring coupled to an inner surface of the chamber; and
an inhalation portion coupled to said proximal end of said chamber, said inhalation portion defining at least one aperture configured to emit powder therethrough;
wherein the inhalation device is configured to have a resistance of at most 0.088 (kPa)^{1/2}/L/min (0.28 (cmH₂O)^{1/2}/L/min) and to provide an emitted dose of at least 85% when the dose of powder is up to 20 mg and when the device is operated at a peak inspiratory flow rate of 25 L/min or less and at an inhalation volume of 0.75 L or less.

The inhalation device may further comprise:
a first casing portion and a second casing portion;
wherein the chamber is a cylindrical chamber and comprises a ring circumferentially coupled to the inner surface of the chamber, and the wall is a straight wall of circular cross-section coupled to said first casing portion; and
wherein the second casing portion is removably coupled to said first casing portion, said second casing portion comprising the inhalation portion which is disposed at the proximal end of said chamber when said first and said second casing portions are coupled, said inhalation portion comprising a hemispheric region defining a plurality of apertures configured to emit powder therethrough.

The standard deviation of the emitted dose may be 10% or less. The at least one aperture may have an area of 0.116 cm² to 0.142 cm² (0.018 in² to 0.022 in²). The ring may have an inner diameter of 0.965 cm to 1.016 cm (0.380 inches to 0.400 inches). The cylindrical chamber may have an inner diameter of 1.016 cm to 1.118 cm (0. 400 inches to 0.440 inches).

The number of vents may be three to five. Each of the plurality of vents may have a width of 0.051 cm to 0.064 cm (0.020 inches to 0.025 inches), a length of 0.495 cm to 0.599 cm (0.195 inches to 0.236 inches) and/or an area of 0.09 cm² to 0.155 cm²(0.014 in² to 0.024 in²).

In one embodiment, said at least one aperture has an aggregate area of 0.129 cm² (0.020 in²), said ring has an inner diameter of 1.016 cm (0.400 inches), said cylindrical chamber has an inner diameter of 1.118 cm (0.440 inches), wherein a number of said plurality of vents is three, each of said plurality of vents has a width of 0.051cm (0.020 inches), and each of said plurality of vents has a length of 0.599 cm (0.236 inches).

In another embodiment, said at least one aperture has an aggregate area of 0.142 cm² (0.022 in²), said ring has an inner diameter of 1.016 cm (0.400 inches), said cylindrical chamber has an inner diameter of 1.118 cm (0.440 inches), wherein a number of said plurality of vents is four, each of said plurality of vents has a width of 0.051 cm (0.020 inches), and each of said plurality of vents has a length of 0.599 cm (0.236 inches).

In a further embodiment, said at least one aperture has an aggregate area of 0.142 cm² (0.022 in²), said ring has an inner diameter of 0.965 cm (0.380 inches), said cylindrical chamber has an inner diameter of 1.016 cm (0.400 inches), wherein a number of said plurality of vents is five, each of said plurality of vents has a width of 0.064 cm (0.025 inches), and each of said plurality of vents has a length of 0.495 cm (0.195 inches).

In one embodiment of the invention, a device for emitting powder is provided. The device includes a first casing portion, and a second casing portion removably coupled to the first casing portion. A cylindrical chamber, defined by a straight wall of circular cross section, is coupled to the first casing portion. The chamber has a proximal end and a distal end. A ring is circumferentially coupled to an inner surface of the chamber. The ring is preferably disposed at approximately a midpoint of the chamber, or, alternatively, disposed adjacent the proximal end of the chamber. The second casing portion includes an emitter portion disposed at the proximal end of the chamber when the first and second casing portions are coupled together. The emitter portion defines at least one aperture configured to emit powder therethrough. The emitter portion is configured as an inhalation portion so that powder is dispersed in the chamber and administered to a user through the inhalation portion. The inhalation portion may be configured as a mouth piece for inhalation through the mouth, or as a nose piece for inhalation through the nose.

In one embodiment, the device of the present invention is configured to cause the emitted powder to be highly dispersed. By "highly dispersed" is meant that the VMGD of the emitted powder is substantially similar to the VMGD of the powder contained in the receptacle. Highly dispersible powders have a low tendency to agglomerate, aggregate or clump together and/or, if agglomerated, aggregated or clumped together, are easily dispersed or de-agglomerated as they emit from an inhaler and are breathed in by the subject. Typically, the highly dispersible particles suitable in the methods of the invention display very low aggregation compared to standard micronized powders which have similar aerodynamic diameters and which are suitable for delivery to the pulmonary system. Properties that enhance dispersibility include, for example, particle charge, surface roughness, surface chemistry, relatively large geometric diameters, and the configuration of the device used to dispense the powder. The volume mean geometric diameter of the powder may be 11.8 µm or less.

The powder is contained in a receptacle that may be disposed in the chamber. Upon puncturing the receptacle, powder is dispersed in the chamber and inhaled from the device.

The device of the present invention may include means for puncturing the receptacle. The means for puncturing can be configured as a staple. Such a staple is preferably configured in a substantially U-shape, having two prongs. In one embodiment of the present invention, each of the prongs has a square cross-section. In another embodiment of the present invention, the substantially U-shaped staple includes a rounded portion and two prongs that define a non-planar inner edge and a non-planar outer edge of the staple, the staple being formed from a rectangular length having two end surfaces and four planar side surfaces that intersect to form four non-planar edges. The inner edge of the staple is configured to be one of the non-planar edges, and the outer edge of the staple is the non-planar edge that is opposite that non-planar edge. Each end surface is an angled diamond-shaped surface. In a preferred embodiment, each end surface has a top point at an apex of the inner edge, and a bottom point at an apex of the outer edge, each top point forming a cutting point for one of the prongs.

In another embodiment, the puncturing means can be configured as a substantially longitudinal prong comprising a puncturing surface on the distal end of the prong, a primary cutting surface running from the proximal end to the distal end of the prong and terminating at the puncturing surface, and a substantially planar face opposite to the primary cutting edge and running from the proximal end to the distal end of the prong. Another embodiment of the puncturing means comprises a substantially longitudinal prong comprising a puncturing surface on the distal end, a primary cutting surface terminating at the puncturing surface, and a face opposite to the primary cutting edge, wherein the prong is configured to create an opening in a wall by forming a hanging chad in the wall, the hanging chad having a free end formed by the puncturing surface and the primary cutting edge and a hinge coupled to the wall formed by the face. In another embodiment, the prong is configured to form a hanging chad in a wall of the receptacle having a longitudinal axis substantially parallel to the prong and a minor axis substantially perpendicular to the longitudinal axis, the hanging chad being opened to an angle of at least 30 to 45 degrees with respect to the minor axis of the receptacle. In another embodiment the prong is configured so that at least 3/4 of the length of the prong can be inserted into a receptacle without breaking off chads in the receptacle.

In each of these embodiments, the prong preferably has an angled surface at the distal end, the surface having a distal end terminating at the puncturing surface and a proximal end terminating at the substantially planar face. In addition, the prong preferably is tapered, so that its distal end is smaller than its proximal end, to facilitate removing the prong from the receptacle. The prong also preferably has a plurality of longitudinal faces and a plurality of longitudinal edges running from the proximal end to the distal end of the prong. In one embodiment, the cross section of the prong is a pentagon. In a related embodiment, the width of the substantially planar face may be very small and the four longitudinal faces may be substantially at right angles to each other so that the prong has substantially a diamond shaped cross section. In another embodiment, the cross section of the prong is a triangle.

In another embodiment, the puncturing means comprises one or more of the longitudinal prongs coupled to a base, preferably in a U-shape. In another aspect of the invention, any of these embodiments of the longitudinal prongs may be coupled to the device for administering powder.

A method for dispensing powder by inhalation may comprise
providing a powder inhalation device, the device comprising
a first casing portion,
a cylindrical chamber, defined by a straight wall of circular cross-section, coupled to said first casing portion, said chamber having a proximal end and a distal end and configured to receive a receptacle therein, said chamber comprising a ring circumferentially coupled to an inner surface of said chamber, and
a second casing portion removably coupled to said first casing portion, said second casing portion comprising an inhalation portion disposed at the proximal end of said chamber when said first and said second casing portions are coupled, said inhalation portion comprising a hemispheric region defining a plurality of apertures configured to emit powder therethrough;
puncturing the receptacle to allow release of powder into said chamber; and
dispersing powder through inhalation of the powder through said inhalation portion.

The inhaling step may be carried out by inhaling the powder through a mouthpiece into a user's mouth. Alternatively, the inhaling step may be carried out by inhaling the powder through a nose piece into a user's nose.
An indicating device may comprise a body disposed within a casing and reversibly moveable between a first and a second position, an indicator moveable between a rest position and an indicating position, and a means for coupling the body and the indicator, wherein upon a first movement of the body from the first position to the second position, the means for coupling couples the body and the indicator, and upon a second movement of the body from the second position to the first position, the indicator moves from the rest position to the indicating position.

An alternative indicating device may comprise a body disposed within a casing and reversibly moveable between a first position and a second position, an indicator reversibly moveable between a rest position and an indicating position, a lip coupled to the indicator and a flange coupled to the body for engaging the lip, wherein upon a first movement of the body from the first position to the second position, the flange engages the lip, and upon a second movement of the body from the second position to the first position, the engagement of the lip and the flange causes the indicator to move from the rest position to the indicating position.

The invention further encompasses one of the previously described devices comprising a means for indicating readiness of the device for emitting powder. The means for indicating readiness of the device for emitting powder may comprise one of the previously described indicating devices.

A method for indicating the readiness of a device for emitting a medicament may comprise:
a device for dispensing a medicament, the device comprising a casing comprising at least one aperture configured to emit powder therethrough, a body coupled to said casing and reversibly moveable between a first position and a second position, and an indicator coupled to said casing and reversibly moveable between a rest position and an indicating position;
applying an axial force to said body to move said body from said first position to said second position, which readies the powder for dispensing and couples said body to said indicator;
releasing said axial force from said body to allow said body to move from said second position to said first position, which moves said indicator to said indicating position; and
dispensing the medicament from said device.

A method for indicating that a device for dispensing a medicament has been used may comprise:
providing a device for dispensing a medicament, the device comprising a casing comprising at least one aperture configured to emit a medicament therethrough, a body coupled to said casing and reversibly moveable between a first position and a second position, and an indicator coupled to said casing and reversibly moveable between a rest position and an indicating position;
applying an axial force to said body to move said body from said first position to said second position, which couples said body to said indicator;
dispensing the medicament from the device;
releasing said axial force from said body to allow said body to move from said second position to said first position, which moves said indicator to said indicating position to indicate that the device has been used.

### Features and Advantages

One feature of the present invention is that it provides, in a low resistance inhaler with a highly dispersed powder, high emitted doses that are consistently reproducible over a range of peak inspiratory flow rates, inhalation volumes and dosage quantities. Advantageously, the present invention improves and optimizes the emitted dose at low peak inspiratory flow rates, low inhalation volumes, and high dose ranges. A particularly advantageous feature of the present invention is its ability to operate at low peak inspiratory flow rates, such as would be associated with a child, an elderly person, or a person with a respiratory disease, such as chronic obstructive pulmonary disease (COPD).

The means for puncturing used in certain embodiments of the device is less expensive to manufacture than conventional piercing devices. Advantages of the injection molding manufacturing process used for the puncturing means include reliability, reproducibility, and design flexibility, such as the ability to make a wide variety of shapes and sizes of longitudinal prongs. For example, larger longitudinal prongs of the present invention can create larger openings in the receptacles than conventional piercing devices, which allows for higher emitted doses at low peak inspiratory flow rates, low volumes, and high dosage quantities. Another advantage of the present invention is that at least one configuration of the puncturing means facilitates forming a hanging chad in the wall of the receptacle, with an opening of at least 30 to 45 degrees, to facilitate more efficient removal of powder from the receptacle and, thus, higher emitted doses than could be achieved with conventional piercing devices. Moreover, the means for puncturing of the present invention advantageously provides improved puncturing performance since less force is needed to puncture the receptacles, and fewer failures result than with conventional piercing devices. Yet another advantage is that the prongs are shaped for easy removal from the receptacle without breaking off the hanging chad formed in the wall of the receptacle.

Another advantage of the preferred means for puncturing is an improvement to the emitted dose rate of the inhaler. In one aspect of the invention, the puncturing means improves the powder flow from the receptacle by increasing the size of the holes in the receptacle. In another aspect of the invention, the puncturing means improves the peak inspiratory flow rate by opening a hanging chad in the wall of the receptacle to an angle of at least 30 to 45 degrees with respect to the wall of the receptacle. Consequently, the emitted dose of the powdered medicament delivered to a patient will be independent of how fast the patient breathes, thereby ensuring that a consistent dose of medicament is delivered each time. Another advantageous feature of the present invention is the accuracy of medicament dosage delivered thereby. Since only one dosage of medication is present in the inhaler during each use, the possibility of overdose is eliminated, and the medicament need not be metered prior to delivery. A patient may simply inhale all medicament present in the device. Yet another advantage is the design of the puncturing means allows for a greater range of puncturing depths without breaking off the chads formed in the receptacle, allowing for greater optimization of the inhaler.

Because the present invention operates only under the inhalative power of the patient, the inhaler carries the additional advantage that no accessory device, such as a compressed air cylinder or other propellant, needs to be used in conjunction with the present invention.

Another advantage of the present invention is that during inhalation, the medicament is subjected to mixing in the dispersion chamber. This helps to ensure that the medicament exiting the inhaler and entering the patient's respiratory system is in the form of a fine dry powder, facilitating medicament deposition in the lungs. In addition, inhalation of finer powders is typically more comfortable for the patient.

An advantage of the present invention is that it can be used with individuals who cannot breathe hard, such as a child, an elderly person, or a person suffering from a respiratory disease, such as asthma, or individuals who are sleeping or in a coma.

Another advantage of the apparatus of the present invention is that it is reusable. To reuse, a patient removes the emptied receptacle, and replaces it with a fresh receptacle filled with the proper dose of medicament.

Another advantage of the present invention is that in certain embodiments, it includes a means for indicating when a device for emitting powder is ready for inhalation. Such a means for indicating informs the user when the device is ready for use and/or when the device needs to be refilled or discarded. For example, the means for indicating could be used with a device for emitting fluticasone propionate (used to treat asthma) to indicate that the device is ready for inhalation. Alternatively, the means for indicating could be used with an epinephrine pen for treating allergies to indicate that the pen has been used. In addition, the means for indicating preferably makes an audible click so that a user will know when the device has been properly actuated. Also, the means for indicating is easy to manufacture and use.

### Brief Description of the Figures

The present invention is described with reference to the accompanying drawings. In the drawings, like reference numbers indicate identical or functionally similar elements.
**FIG. 1** is a front view of one embodiment of a device of the present invention;
**FIG. 2** is a cross-section of the device shown in FIG. 1 along line 2-2;
**FIG. 3** is an enlarged partial cross-section of one embodiment of a dispersion chamber of the present invention;
**FIG. 4** is an enlarged partial cross-section of another embodiment of a dispersion chamber of the present invention showing one location for a ring in the dispersion chamber;
**FIG. 5** is an enlarged partial cross-section of another embodiment of a dispersion chamber of the present invention showing another location for a ring in the dispersion chamber;
**FIG. 6** is an enlarged partial cross-section of another embodiment of a dispersion chamber of the present invention showing another location for a ring in the dispersion chamber;
**FIG. 7A** is a top view of a preferred embodiment of a staple suitable for use with the device of the present invention;
**FIG. 7B** is a front view of the embodiment shown in FIG. 7A;
**FIG. 7C** is a side view of the embodiment shown in FIG. 7A;
**FIG. 7D** is an isometric view of the embodiment shown in FIG. 7A;
**FIG. 8** shows the puncture obtained with the staple shown in FIGS. 7A through 7D;
**FIG. 9A** shows a partial view of another embodiment of a staple suitable for use with the device of the present invention;
**FIG. 9B** illustrates the puncture obtained with the staple shown in FIG. 9A;
**FIG. 10** is a bar graph illustrating emitted dose at peak inspiratory flow rates of 20 L/min (left bar), 40 L/min (center bar), and 60 L/min (right bar) for four dispersion chamber configurations;
**FIG. 11** is a bar graph illustrating emitted dose at low peak inspiratory flow rates for devices with varying numbers of vents;
**FIG. 12** is a bar graph showing a comparison of mass fraction distributions obtained for 6 mg (left bar) and 50 mg (right bar) fill weights;
**FIG. 13** is a graph showing glucose levels (mg/dL) in beagle dogs after administration of insulin using an aerosol generator and a device of the present invention with the low ring configuration substantially as shown in FIG. 4;
**FIG. 14** is a bar graph illustrating the percentage emitted dose as a function of air volume; and
**FIG. 15** is an exploded cross-sectional view of an alternate embodiment of a device of the present invention.
**FIG. 16A** is a perspective view of an alternative embodiment of a puncturing device suitable for use with the present invention.
**FIG. 16B** is a front view of the puncturing device shown in FIG. 16A.
**FIG. 16C** is a side view of the puncturing device shown in FIG. 16A.
**FIG. 16D** is a top view of the puncturing device shown in FIG. 16A.
**FIGS. 17A-17C** are schematic diagrams of one of the prongs of the puncturing device shown in FIGS. 16A-D being used to puncture a receptacle and create a hanging chad therein.
**FIG. 18** is a front cross-sectional view of an alternative embodiment of the device for administering powder comprising a means for indicating the readiness of the device.
**FIGS. 19A-19C** are enlarged partial cross-sectional views of a preferred embodiment of the means for indicating readiness of the device.

### Detailed Description of the Preferred Embodiments

### Overview

The present invention provides an apparatus for facilitating release of powder that is contained in a receptacle. As used herein, the term "receptacle" includes but is not limited to, for example, a capsule, blister, film covered container well, chamber, and other suitable means of storing a powder known to those skilled in the art. The present invention will be described below in the context of an apparatus for dispensing dry powder medicaments for inhalation by a patient. However, it should be apparent to one skilled in the art that the invention is not limited to such an exemplary embodiment, and could be used for other purposes.

As will be described in more detail below, an apparatus of the present invention is an inhaler that includes a chamber. In one embodiment, the chamber is configured to receive the receptacle containing the medicament. To improve the emptying of the receptacle and provide a higher reproducible emitted dose, the chamber includes a ring circumferentially coupled to an inner surface of the chamber. The ring is preferably disposed at approximately a midpoint of the chamber, or alternatively, adjacent the proximal end of the chamber. In proper use, air will exit the inhaler carrying a full dose of medicament in the form of a fine, dry powder.

The inhalation device of the present invention is configured to have a resistance of at most 0.28 (cm H₂O)^{1/2}/L/min (0.088 (kPa)^{1/2}/L/min) and to provide an emitted dose or at least 85% when the dose of powder is up to 20 mg and when the device is operated at a peak inspiratory flow rate of 25 L/min or less and at an inhalation volume of 0.75 L or less.

The inhaler of the present invention can be configured with a means for puncturing the receptacle that improves puncturing performance, particularly with brittle receptacle material. In one preferred embodiment, the means for puncturing the receptacle of the present invention is configured as a substantially U-shaped staple with two prongs, each prong having a sharp point and two cutting edges. In one such embodiment, each prong has a square cross-section, with the staple material being bent around a face so that the innermost part of the U-shaped staple is flat. In another such embodiment, the staple material is rotated 45 degrees so that it is bent around an edge so that the innermost part of the U-shaped staple is an edge. In such an embodiment, the end surface of each prong is an angled diamond-shaped surface.

In another embodiment, the means for puncturing the receptacle is configured as a substantially longitudinal prong comprising a puncturing surface on the distal end, a primary cutting surface running from the proximal end to the distal end of the prong and terminating at the puncturing surface, and a substantially planar face opposite to the primary cutting edge and running from the proximal end to the distal end of the prong. The prong preferably has an angled surface at the distal end, the angled surface having a distal end terminating at the puncturing surface and a proximal end terminating at the substantially planar face. In addition, the prong is preferably tapered so that the distal end is smaller than the proximal end, to facilitate removing the prong from a receptacle. The prong also preferably has a plurality of longitudinal faces and a plurality of longitudinal edges running from the proximal end to the distal end of the prong.

The prong is configured to create an opening in a wall by forming a hanging chad in the wall, the hanging chad having a free end formed by the puncturing surface and the primary cutting edge and a hinge coupled to the wall formed by the face. In a preferred embodiment, the prong is configured.to open the hanging chad to an angle of at least 30 to 45 degrees between the minor axis of the receptacle and the hanging chad, wherein the minor axis is substantially perpendicular to a longitudinal axis of the receptacle, which is substantially parallel to the longitudinal prong.

The device of the present invention is used to dispense powder by inhalation. As will be discussed in greater detail below, a user operates the device to puncture the receptacle to disperse powder in the chamber, and inhales the powder through the inhalation portion. The present invention further encompasses a means for indicating readiness coupled to a device for administering powder.

### Inhaler and Associated Method of the Present Invention

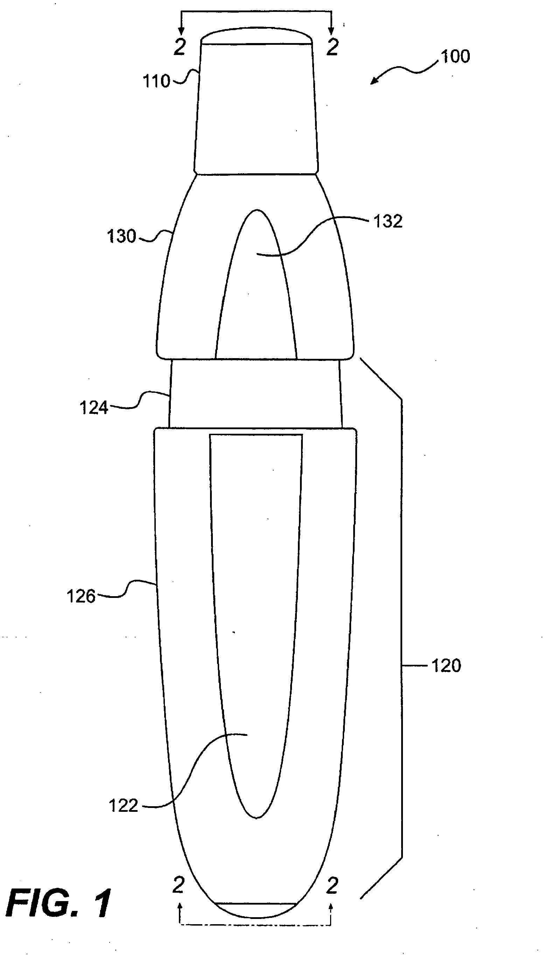

A front view of one embodiment of an inhalation device **100** of the present invention is shown in FIG. 1. The rear view of device **100** is substantially identical to the front view. Device **100** includes a first or lower casing portion **120** and a second or upper casing portion **130** removably coupled to first casing portion **120.** Upper casing portion **130** and lower casing portion **120** include a flattened region **132** and **122,** respectively, for ease of gripping the casing for use by a patient. Lower casing portion **120** preferably includes an outer casing **126** and an inner casing **124** movably received within outer casing **126.** A removable cap **110** is provided at the user or inhalation end of the device.

Preferred materials for device **100** include Food and Drug Administration (FDA) approved, USP tested plastics. Preferably, device **100** is manufactured using an injection molding process, the details of which would be readily apparent to one skilled in the art.

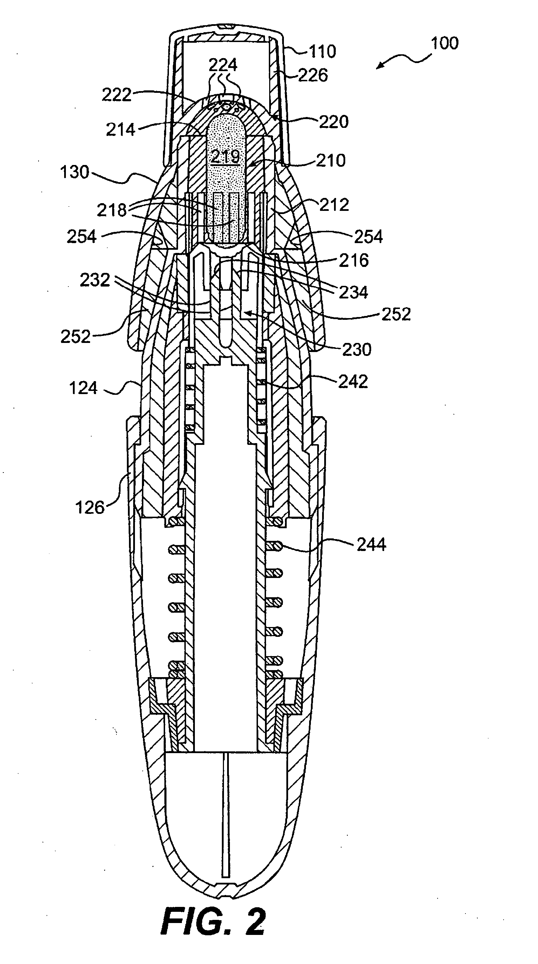

FIG. 2 is a cross-section of device **100** shown in FIG. 1 along line 2-2. As shown in FIG. 2, device **100** includes an inhalation or emitter portion **220.** Inhalation portion **220** comprises a hemispheric region **222** that defines a plurality of apertures **224.** It should be understood that the present invention is not limited to a particular number of apertures **224,** and can be configured such that at least one aperture **224** is provided. An inhalation piece **226** is provided to allow for inhalation of the medicament by a user. Inhalation piece **226** can be configured as a mouth piece for inhalation through a user's mouth. Alternatively, inhalation piece **226** can be configured as a nose piece for inhalation through a user's nose.

Device **100** includes a cylindrical chamber **210** that is defined by a straight wall **212** of circular cross-section. Chamber **210** has a proximal end **214** and a distal end **216.** A plurality of vents **218** are defined by wall **212,** and are configured for introducing air into chamber **210** to disperse powder released from a capsule **219.** It should be understood that the present invention is not limited to a particular number of vents **218,** and can be configured such that at least one vent **218** is provided. Powder released from capsule **219** is dispersed in chamber **210** and inhaled through apertures **224** and inhalation piece **226** by the user.

Receptacles other than capsules are used, such as blisters and film covered container wells as is known in the art. In one embodiment, the volume of the receptacle is at least about 0.37 cm³. In another embodiment, the volume of the receptacle is at least about 0.48 cm³. In yet another embodiment, the receptacles have a volume of at least about 0.67 cm³ or 0.95 cm³. In one embodiment of the invention, the receptacle is a capsule designated with a capsule size 2, 1, 0, 00, or 000. Suitable capsules can be obtained, for example, from Shionogi (Rockville, MD). Blisters can be obtained, for example, from Hucck Foils, (Wall, NJ).

The receptacle encloses or stores particles, also referred to herein as powders. The receptacle is filled with particles in a manner known to one skilled in the art For example, vacuum filling or tamping technologies may be used. Generally, filling the receptacle with powder can be carried out by methods known in the art. In one embodiment of the invention, the particle or powder enclosed or stored in the receptacle have a mass of at least about 5 milligrams (mg). In another embodiment, the mass of the particles stored or enclosed in the receptacle is at least about 10 mg, and up to approximately 50 mg. In a preferred embodiment, the mass of the particles is approximately 20 mg.

In one embodiment of the present invention, particles used with the device have a tap density of less than about 0.4 g/ cm³. Particles having a tap density of less than about 0.4 g/cm³ are referred to herein as "aerodynamically light". In a preferred embodiment, the particles have a tap density of near to or less than about 0.1 g/cm³. Tap density is a measure of the envelope mass density characterizing a particle. The envelope mass density of particles of a statistically isotropic shape is defined as the mass of the particle divided by the minimum sphere envelope volume within which it can be enclosed. Features that can contribute to low tap density include irregular surface texture and hollow or porous structure. Particularly preferred particles and powders are described in U.S. Patent Nos. 6,136, 295, 5,985,309, 5,874,064, 5,855,913, and 6,858,199.

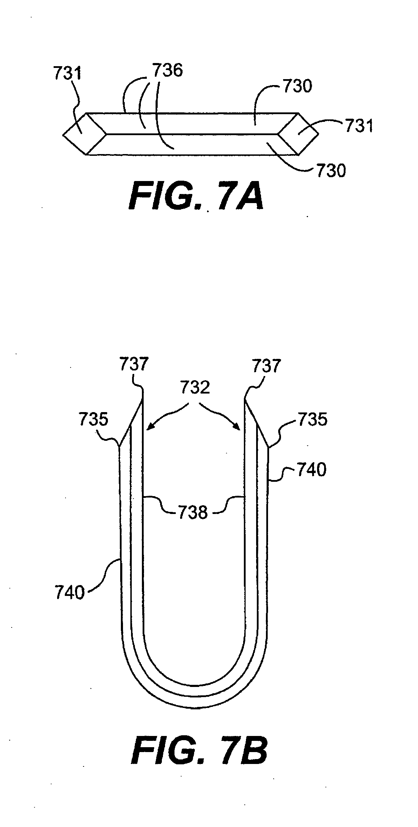

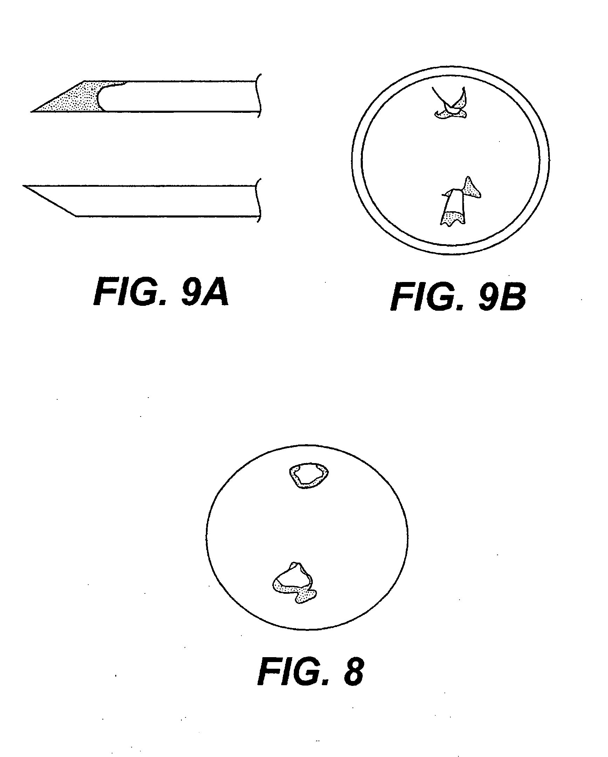

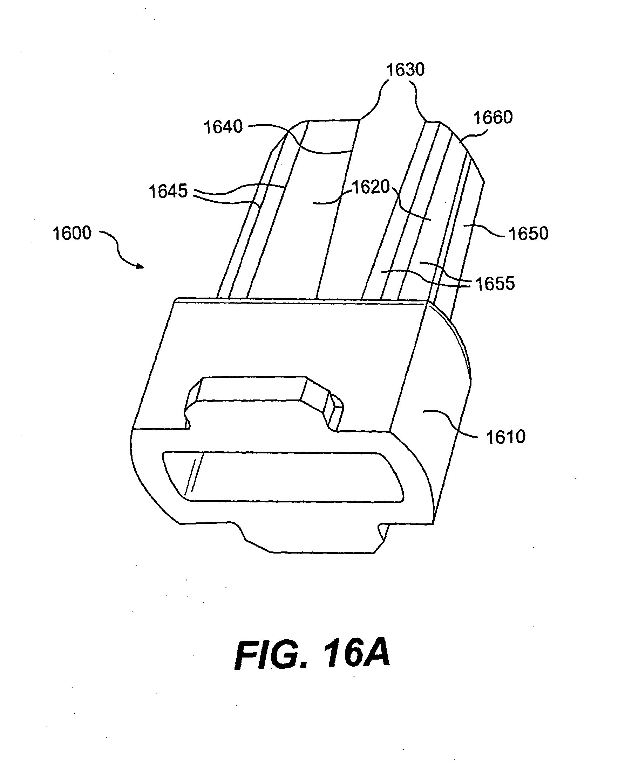

Device 100 includes a means for puncturing **230** that is used to puncture capsule **219** to release powder contained therein into chamber **210.** In the embodiment shown in FIG. 1, means for puncturing **230** is configured as a substantially U-shaped staple having two prongs **232.** In this embodiment, each of prongs **232** is configured with a square cross-section **234,** thereby providing a sharp point and two cutting edges. This will be discussed in more detail below with respect to FIGS. 9A and 9B. As discussed in more detail below, device **100** could alternatively be configured with the means for puncturing shown in FIGS. 7A through 7D. Also, device **100** could alternatively be configured with the means for puncturing shown in FIGS. 16A through 16D. As can be readily appreciated by one skilled in the art, the present invention is not limited to these means for puncturing the capsule, described in detail below. For example, one, or a plurality of, straight needle-like implements could be used. Preferably, the means for puncturing is configured to puncture at least two holes in the capsule.

Means for puncturing **230** is preferably configured to be movable between a non-puncturing position (as depicted in FIG. 1) and a puncturing position. In the puncturing position, prongs **232** pierce or puncture capsule **219** to make holes therein. In a preferred embodiment, a means for biasing is provided that biases the means for puncturing **230** in the non-puncturing position. In the embodiment shown in FIG. 2, the means for biasing is configured as a spring **242** that biases the substantially U-shaped staple in the non-puncturing position.

As noted with respect to FIG. 1, device **100** includes inner casing **124** and outer casing **126.** As shown in FIG. 2, a spring **244** is disposed in lower casing portion **120** that biases inner casing **124** in an outward position. Upon compression of spring **244,** inner casing **124** moves from the outward position to an inward position, thereby drawing lower casing portion **120** toward upper casing portion **130.** Compression of spring **244** also causes compression of spring **242,** thereby causing means for puncturing **230** to move to the puncturing position. Upon release of compression, springs **242** and **244** return to their biased state, thereby returning means for puncturing **230** to its non-puncturing position, and inner casing **124** to its outward position.

A pair of flanges **252** is disposed on first casing portion **120.** A pair of grooves **254** is disposed on second casing portion **130** so that flanges **252** can be received within grooves **254** to thereby couple the first and second casing portions. Preferably, the first and second casing portions are coupled with a friction-fit engagement. A friction-fit engagement can be achieved using the groove and flange arrangement depicted in FIG. 2. Other alternative configurations for a friction-fit engagement would be readily apparent to one skilled in the art.

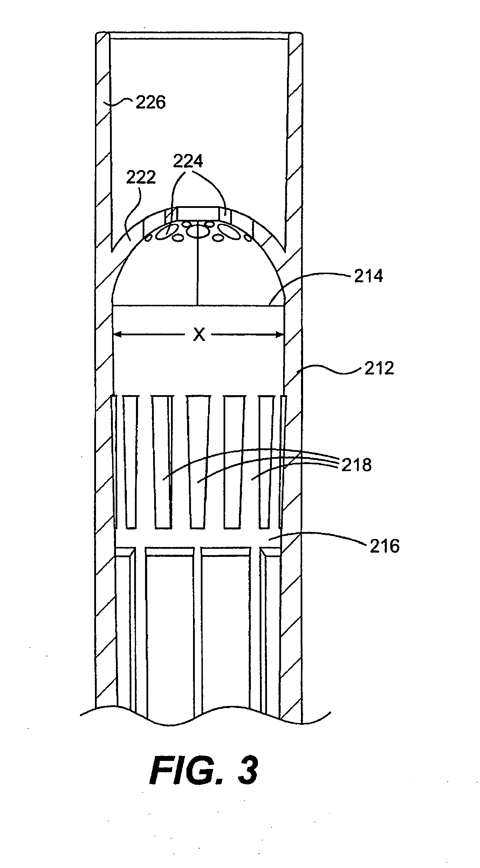

FIG. 3 is an enlarged partial cross-section of one embodiment of chamber **210.** In the embodiment shown in FIG. 3, chamber **210** does not contain a ring disposed on an inner surface, and an inner diameter of chamber **210** is depicted as "X". Such a configuration may be referred to herein as a "straight" chamber configuration.

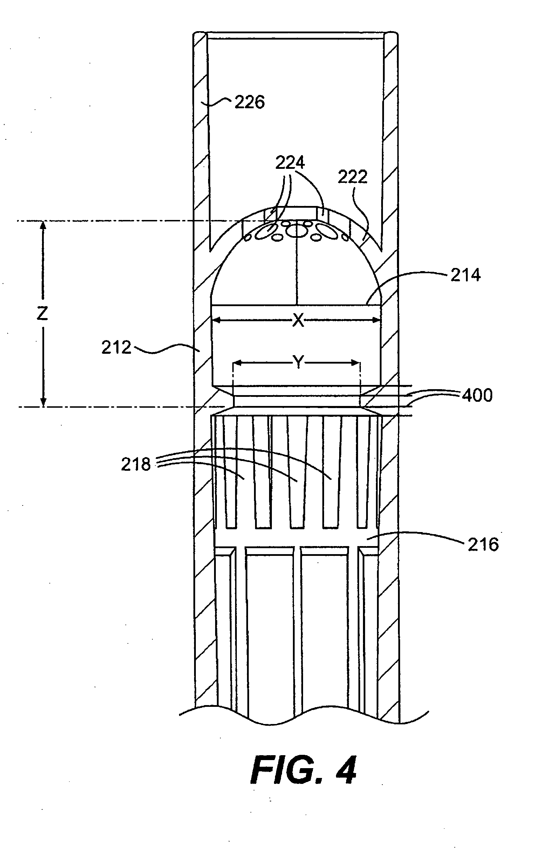

FIG. 4 is an enlarged partial cross-section of another embodiment of chamber **210.** In the embodiment shown in FIG. 4, a ring **400** is circumferentially coupled to an inner surface of chamber **210.** An inner diameter of ring **400** is depicted as "Y", and is less than inner diameter X of chamber **210.** In the embodiment shown in FIG. 4, ring **400** is disposed at approximately a midpoint of chamber **210.** Such a configuration may be referred to herein as a "low" ring position or "low" chamber configuration. As shown in FIG. 4, in the low ring position, ring **400** is disposed adjacent vents **218.** The ring position is measured by the distance from the top of hemispheric region **222** to the bottom edge of ring **400.** This distance is depicted as "Z". The following dimensions are provided as exemplary dimensions of a device of the present invention. It should be understood by one skilled in the art that the present invention is not limited to the dimensions provided herein, or to any particular dimensions. In one embodiment of the chamber **210** shown in FIG. 4, diameter X is 1.19 cm (0.47 in.), diameter Y is 0.97 cm (0.38 in.), and distance Z is 1.25 cm (0.49 in.).

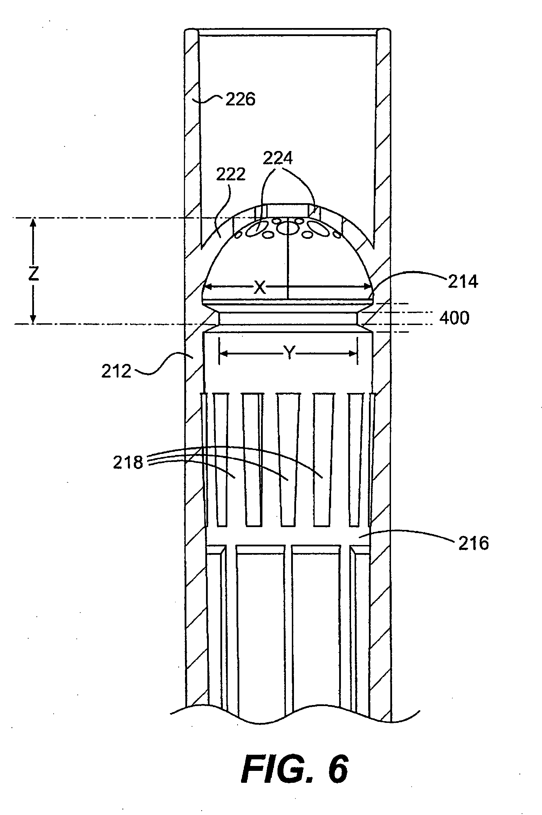

FIG. 6 is an enlarged partial cross-section of another embodiment of chamber **210.** In the embodiment shown in FIG. 6, ring **400** is circumferentially coupled to an inner surface of chamber **210.** An inner diameter of ring **400** is depicted as "Y", and is less than inner diameter X of chamber **210.** In the embodiment shown in FIG. 6, ring **400** is disposed adjacent the proximal end of chamber **210.** Such a configuration may be referred to herein as a "high" ring position or a "high" chamber configuration. The ring position is measured by the distance from the top of hemispheric region **222** to the bottom edge of ring **400.** This distance is depicted as "Z". The following dimensions are provided as exemplary dimensions of a device of the present invention. It should be understood by one skilled in the art that the present invention is not limited to the dimensions provided herein, or to any particular dimensions. In one embodiment of the chamber **210** shown in FIG. 4, diameter X is 1.19 cm (0.47 in.), diameter Y is 0.97 cm (0.38 in.), and distance Z is 0.74 cm (0.29 in.).

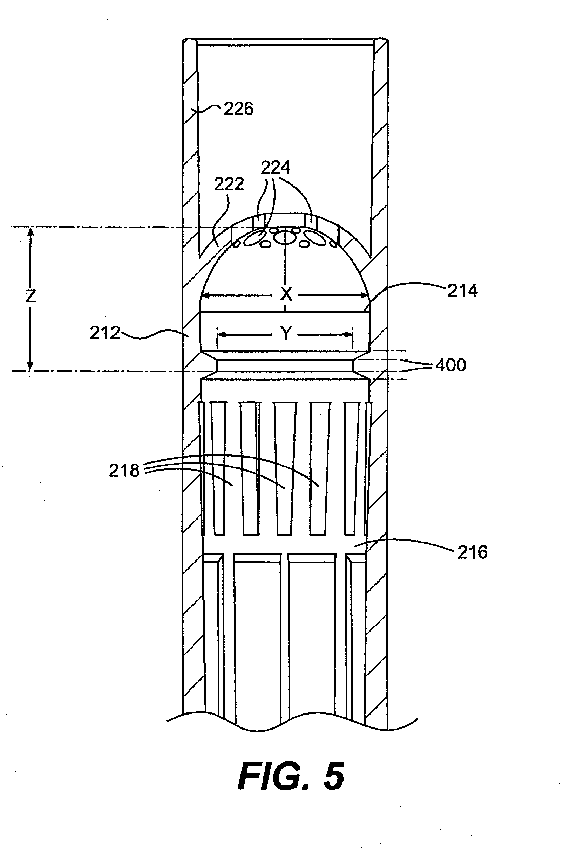

FIG. 5 is an enlarged partial cross-section of another embodiment of chamber **210.** In the embodiment shown in FIG. 5, ring **400** is circumferentially coupled to an inner surface of chamber **210.** An inner diameter of ring **400** is depicted as "Y", and is less than inner diameter X of chamber **210.** In the embodiment shown in FIG. 5, ring **400** is disposed between the low ring position of FIG. 4 and the high ring position of FIG. 6. Such a configuration may be referred to herein as a "mid" ring position or "mid" chamber configuration. The ring position is measured by the distance from the top of hemispheric region **222** to the bottom edge of ring **400.** This distance is depicted as "Z". The following dimensions are provided as exemplary dimensions of a device of the present invention. It should be understood by one skilled in the art that the present invention is not limited to the dimensions provided herein, or to any particular dimensions. In one embodiment of the chamber **210** shown in FIG. 4, diameter X is 1.19 cm (0.47 in.), diameter Y is 0.97 cm (0.38 in.), and distance Z is 0.99 cm (0.39 in.).

In one embodiment of the present invention, ring **400** is integral with chamber **210.** In such an embodiment, ring **400** and chamber **210** are formed as a unit, such as through an injection molding, extrusion or a casting process. In another embodiment of the present invention, ring **400** is attached to the inner surface of chamber **210** in a manner known to those skilled in the art, such as through the use of glue or other type of adhesive, or by using an attaching device such as a pin or screw, etc. Preferably, the casing of device **100** is made from a material that can be injection molded, such as a plastic material (preferably FDA approved, USP tested). As would be readily apparent to one skilled in the art, the material is preferably durable, easy to clean, and non-reactive with powder medicaments.

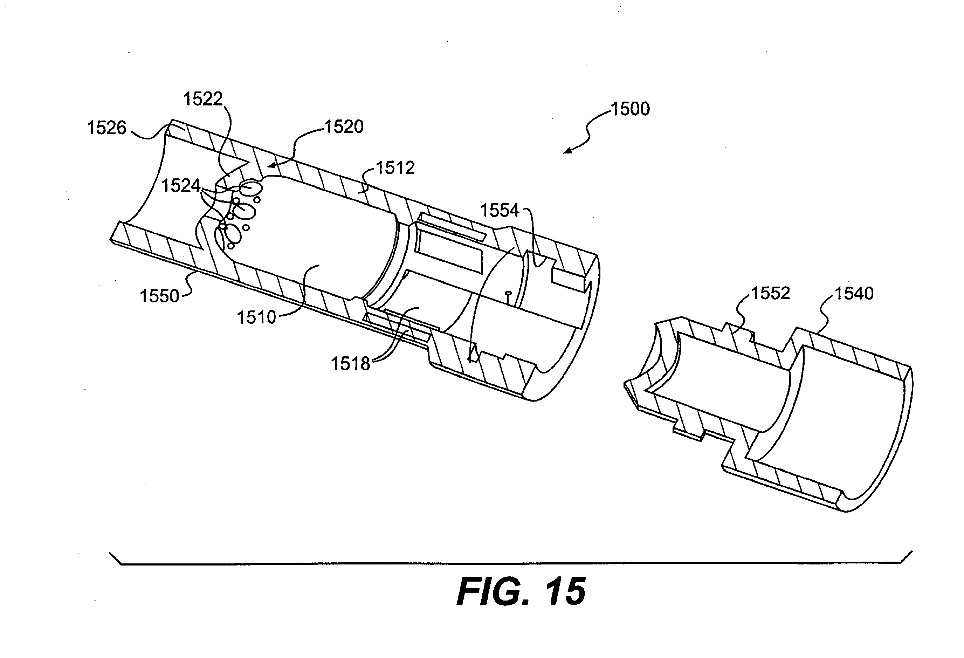

An exploded cross-sectional view of an alternate embodiment of a device **1500** of the present invention is shown in FIG. 15. Device **1500** includes a first or lower casing portion **1540** and a second or upper casing portion **1550** removably coupled to first casing portion **1540.** First and second casing portions **1540** and **1550** are coupled through the use of a flange **1552** and a groove **1554.** Preferred materials for device **1500** include Food and Drug Administration (FDA) approved, USP tested plastics. Preferably, device **1500** is manufactured using an injection molding process, the details of which would be readily apparent to one skilled in the art.

Device **1500** includes an inhalation or emitter portion **1520.** Inhalation portion **1520** comprises a hemispheric region **1522** that defines a plurality of apertures **1524.** It should be understood that the present invention is not limited to a particular number of apertures **1524,** and can be configured such that at least one aperture **1524** is provided. An inhalation piece **1526** is provided to allow for inhalation of the medicament by a user. Inhalation piece **1526** can be configured as a mouth piece for inhalation through a user's mouth. Alternatively, inhalation piece **1526** can be configured as a nose piece for inhalation through a user's nose.

Device **1500** includes a cylindrical chamber **1510** that is defined by a straight wall **1512** of circular cross-section. A plurality of vents **1518** are defined by wall **1512,** and are configured for introducing air into chamber **1510** to disperse powder released from, for example, capsule **219** as illustrated in FIG. 2. It should be understood that the present invention is not limited to a particular number of vents **1518,** and can be configured such that at least one vent **1518** is provided. Powder released from capsule **219** is dispersed in chamber **1510** and inhaled through apertures **1524** and inhalation piece **1526** by the user.

As would be readily apparent to one skilled in the art, device **1500** can be configured with means for puncturing and means for biasing in a manner similar to that described above with respect to the embodiment shown in FIGS. 1 and 2. Means for puncturing are described in more detail below with respect to FIGS. 7A through 7D, 8, 9A-9B, 16A-16D, and 17A-17C. Moreover, device **1500** can be configured with the chamber designs described above with respect to FIGS. 3-6.

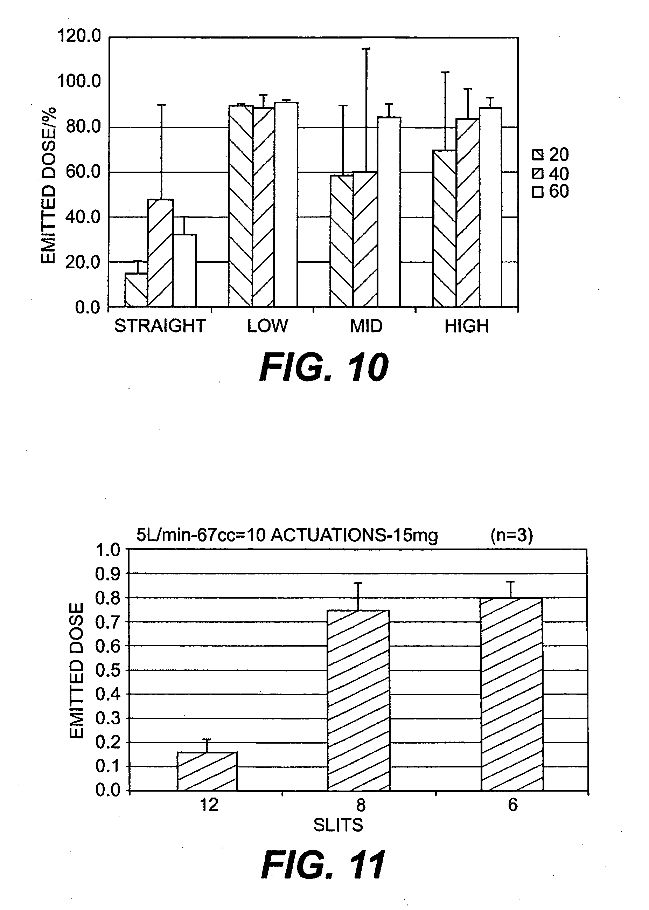

FIG. 10 is a bar graph illustrating emitted dose at peak inspiratory flow rates of 20 L/min (left bar), 40 L/min (center bar), and 60 L/min (right bar) for a total volume of 2L for four dispersion chamber configurations (standard deviations shown; sample size n=3). The peak inspiratory flow rates were measured with a flow meter. The emitted dose measurement involved placing a capsule into four embodiments of the inhaler of the present invention for actuation into an emitted dose (ED) measurement apparatus. The ED apparatus included a powder filter and a filter holder. The powder collected by the ED apparatus was quantified by fluorescence spectrophotometry. The straight configuration is shown in FIG. 3; the low configuration is shown in FIG. 4; the mid configuration is shown in FIG. 5; and the high configuration is shown in FIG. 6. As can be seen from FIG. 10, each of the low, mid, and high configurations demonstrated a higher emitted dose at each of the three flow rates than the straight (no ring) configuration. Thus, the ring configuration of the present invention provides an improvement over conventional chamber designs without a ring, such as those shown in the '819 and '385 patents. At each of the flow rates shown in FIG. 10, the low configuration produced a higher emitted dose and a lower standard deviation than the mid and high configurations.

FIG. 11 is a bar graph illustrating emitted dose at low peak inspiratory flow rates for devices with varying numbers of vents **218.** The measurements were taken at a flow rate of 5 L/min, with a volume of 67 cc and a 15 mg dosage. As show in FIG. 11, by decreasing the number of vents **218,** the emitted dose increases so that the device of the present invention successfully delivers a high emitted dose at a low peak inspiratory flow rate over multiple (ten) actuations. Thus, the device of the present invention achieves a high emitted dose at low peak inspiratory flow rates that is consistently reproducible with low standard deviation.

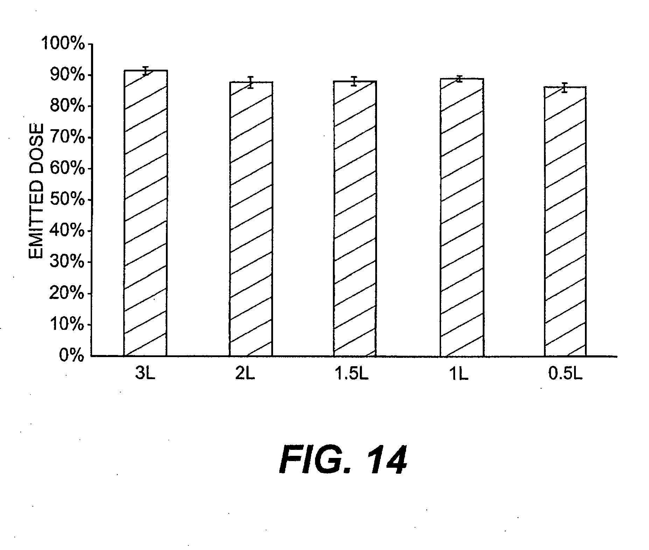

Experiments were conducted to evaluate the emitted dose as a function of air volume drawn through the inhaler. The inhaler was operated at a constant flow rate of 30 L/min for a 5 mg dose. The volume of air through the inhaler was varied by varying the actuation time. Volumes of 0.5, 1.0, 1.5, 2.0 and 3.0 L were investigated. FIG. 14 shows the percentage emitted dose as a function of air volume (n=3, standard deviations shown). The emitted dose remained constant across the range of volumes and was consistently reproducible with low standard deviation.

In the embodiments having the inner diameter X of chamber **210** of 0.47 in. and the inner diameter Y of ring **400** of 0.97 cm (0.38 in.), the ratio of the inner diameter of the ring to the inner diameter of the chamber is about 0.8. By modifying the inner diameters of the ring and the chamber, it is possible to optimize the emitted dose at varying flow rates. As reported in Annals of the ICRP, Human respiratory tract model for radiological protection, 24 (1-3), Elsevier Science, Inc., New York, 1994, the peak inspiratory flow rate for a tidal breathing seated adult male is 300 mL/s (18 L/min) for a volume of 750 mL. In one embodiment of a device of the present invention optimized for low peak inspiratory flow rates, inner diameter X of chamber **210** is 0.84 cm (0.33 in.) and inner diameter Y of ring **400** is 0.765 cm (0.30 in.). In such an embodiment, the ratio of the inner diameter of the ring to the inner diameter of the chamber is about 0.9. Preferably, the ratio of the inner diameter of the ring to the inner diameter of the chamber is about 0.9 or less.

The device of the present invention can also be optimized for varying dosage ranges. One way to do so is to vary the dimensions of chamber **210** to accommodate varying sizes of capsules. For example, a chamber having an inner diameter X of 0.33 in., inner diameter Y of 0.76 cm (0.30 in.), and distance Z of 1.45 cm (0.57 in.) can be used with size 2 and size 00 capsules. It should be readily apparent to one skilled in the art that chamber **210** can be scaled to accommodate varying capsule sizes, and to accommodate those capsule sizes at varying peak inspiratory flow rates.

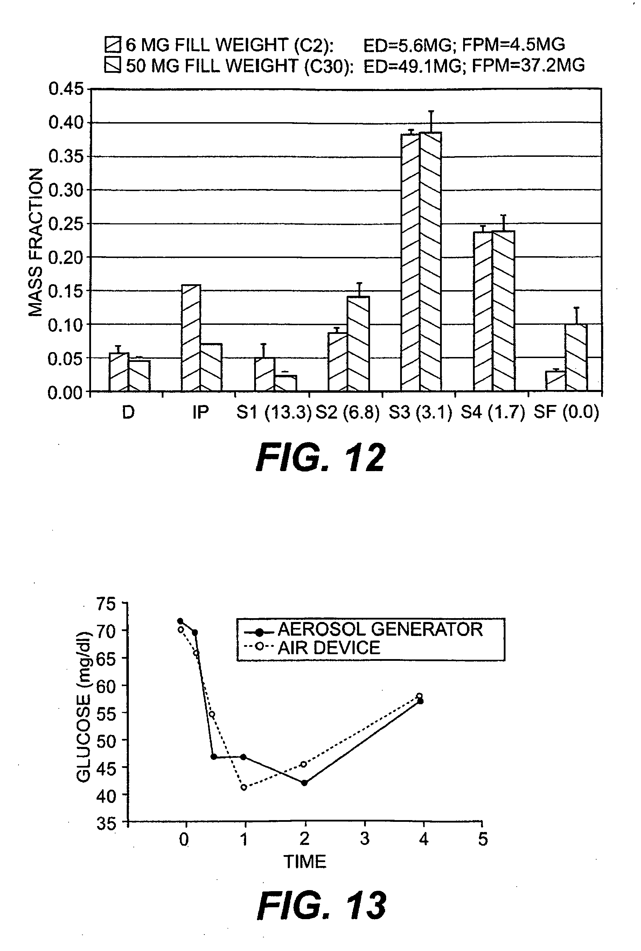

The device of the present invention can be used with varying dosage ranges. A highly dispersible powder was prepared and loaded into capsules to obtain a large pre-metered dose (50 mg) and a smaller pre-metered dose (6 mg). The particle size characteristics of the powder were as follows: VMGD=10.6µm; ρ=0.11 g/cc; and Da=3.5 µm, where VMGD is the volume mean geometric diameter, ρ is the powder density, and Da is the mean aerodynamic diameter. The aerodynamic particle size distributions were characterized using a multistage liquid impinger that extracted air at 60 L/min after actuating the inhaler device (D). As shown in FIG. 12, the mass fraction was measured at D, the induction port (IP) of the impactor, stages S1-S4, and the filter cutoff (SF). Size 2 capsules were used for the 6 mg dose and size 000 capsules were used for the 50 mg dose. FIG. 12 shows the results comparing the two particle size distributions obtained for the 6 mg (left bar) and 50 mg (right bar) doses. "ED" used on the graph refers to emitted dose, and FPM used on the graph refers to fine particle mass (estimate of the mass that would deposit in the lungs). The fine particle fraction <6.8 µm relative to the total dose (FPF_{TD} <6.8 µm) for the 6 and 50 mg doses were 74.4% and 75.0%, respectively. Similar aerodynamic particle size distributions were obtained for both doses.

FIG. 13 is a graph showing glucose (mg/dL) in beagle dogs after administration of human insulin using an aerosol generator and a device of the present invention with the low ring configuration substantially as shown in FIG. 4. The generator is a device with proven ability for forming a respirable aerosol that results in deposition of powder in dog lungs. Metered powder is presented to a chamber where the powder is dispersed by a high velocity jet of air. The dispersed powder is directed toward a baffle to separate large agglomerates before inhalation by the dog. The pharmakodynamic profile shown in FIG.13 confirms that the device of the present invention produces a pattern of powder deposition similar to the aerosol generator.

The dogs were anesthetized for the dosing procedure. A forced maneuver was used with dogs being ventilated at 75% of their vital capacity (approximately 100 cc/s or 6 L/min for a duration of 1 second). A 4 second breath-hold was applied at the end of each inhalation. A physically smaller device was used with the low ring configuration to facilitate administration. The device performed well at the low peak inspiratory flow rate with the anesthetized dogs using the forced maneuver. Based on these results, such a device could be used with a sleeping person or a person having breathing problems, such as from chronic obstructive pulmonary disease (COPD).

As can be seen from the description above, the device of the present invention relies upon the breath of the user to drive the inhalation process, yet the device is configured to work successfully at low peak inspiratory flow rates. As such, the device of the present invention has particular suitability for use with individuals who cannot breath hard, such as a child, an individual with respiratory disease, or individuals who are sleeping or in a coma.

The present invention further encompasses optimizing the configuration of device chamber **210** in order to maintain a low resistance of at most 0.28 (cm H₂O)^{1/2}/L/min (0.088 (kPa)^{1/2}/L/min) and to achieve an emitted dose at least 85% when the receptacle contains a dose of up 10 to 50 mg of powder and when the device is operated at a peak inspiratory flow rate of 25 L/min or less and at an inhalation volume of 0.75 L or less. Experiments were performed on various chamber configurations, using size 00 capsules filled with a 20 mg dose of standard test powder. The various configurations were tested for emitted dose (ED), using known methods described above, at peak inspiratory flow rates ranging from 15 L/min to 25 L/min and at inhalation volumes ranging from 0.25 L/min to 0.75 L/min. In addition, the dispersion of the powder was quantified by measuring the volume mean geometric diameter (VMGD) of the emitted powder, by employing a RODOS dry powder disperser (or equivalent technique) such that at about 1 Bar, particles of the dry powder emitted from the RODOS orifice with geometric diameters, as measured by a HELOS or other laser diffraction system, are less than about 1.5 times the geometric particle size as measured at 4 Bar. In addition, the resistance of each chamber was measured using methods that will be apparent to one of ordinary skill in the art.

The following dimensions of chamber **210** were varied in order to discover the optimal combination: mouthpiece hole area, mouthpiece hole number, chamber diameter (X in FIG. 4), ring diameter (Y in FIG. 4), vent area (the product of vent width, vent height, and vent number), and capsule hole area (the product of the hole area and the number of holes). Initially, it was discovered that it is always desirable to maximize the capsule hole area. Accordingly, the capsule hole area was fixed at 0.084cm² (0.013 square inches). It should be understood that the present invention encompasses other capsule hole areas, especially when used with different sized capsules. It was also determined that the total area of the holes in the mouthpiece was an important factor but that the number of holes in the mouthpiece did not affect the results.

Next, 130 chambers were tested, each having a different combination of mouthpiece hole area, chamber diameter, ring diameter, and vent area. During the testing it was discovered that each of these dimensions have competing effects on the emitted dose, the volume mean geometric diameter, and the resistance of the chamber. For example, increasing the vent area has a positive impact on (i.e., decreases) resistance, but has a negative effect on (i.e., decreases) emitted dose and has a negative effect on (i.e., increases) volume mean geometric diameter. Other dimensions have similar competing effects. In addition, as shown in FIGS. 20A to 20C and discussed in detail below, the vent area and the chamber diameter have combinational effects on the properties of the chamber. Other combinations of dimensions have similar combinational effects.

Of the 130 chambers tested, three preferred embodiments of chambers were identified that achieved the desired characteristics. The pertinent dimensions of each of those chambers is described in Table 1.

**Table 1- Aspects of Preferred Embodiments of Chambers**

| | Chamber F | Chamber H | Chamber I |
|---|---|---|---|
| Resistance (cm H₂O)^{1/2}/L/min (kPa)^{1/2}/L/min) | 0.27 (0,084) | 0.22 (0.070) | 0.19 (0.059) |
| Mouthpiece Hole Area (cm²)(sq.in.) | 0.129 (0.020) | 0.142 (0.022) | 0.142 (0.022) |
| Chamber Diameter (cm)(in.) | 0.118(0.440) | 0.11(0.436) | 0.12(0.440) |
| Ring Diameter (cm) (in.) | 1.016 (0.400) | 0.97 (0.380) | 1.016 (0.400) |
| Vent Area (cm²) (in.) | 0.09 (0.014) | 0.129(0.020) | 0.155 (0.024) |
| Vent Number | 3 | 4 | 5 |
| Vent Width (cm) (in.) | 0.05 (0.020) | 0.06 (0.025) | 0.05 (0.020) |
| Vent Length (cm) (in.) | 0.60 (0.236) | 0.50 (0.195) | 0.60 (0.236) |

Tables 2-4 summarize the emitted dose (ED) (in percent) and dispersion (volume mean geometric diameter (VMGD) in microns)) (with standard deviations in parentheses) achieved with each of these preferred embodiments of chambers, operated with a capsule having a dose of approximately 20 mg and at peak inspiratory flow rates from 15 L/min to 25 L/min and at inhalation volumes from 0.25 L to 0.75 L. The test powder, referred to herein as "standard test powder," was a placebo powder of 84.99 wt% maltodextran, 15 wt% leucine, and 0.01 wt% rhodamine. It had a VMGD of 12 µm measured using the RODOS at 1 bar and an aerodynamic size (volume mean aerodynamic diameter or VMAD) of 3 µm measured using an 8 stage Anderson Cascade Impactor. The goal emitted dose was at least 85%. The goal dispersion for the standard test powder was a VMGD of 11.8 µm or less, although it should be understood that this goal would vary depending on the type of powder used.

**Table 2 - Chamber F**

| Volume → | 0.25 L | | 0.5 L | | 0.75 L | |
|---|---|---|---|---|---|---|
| Flow Rate | VMGD | ED | VMGD | ED | VMGD | ED |
| 15 L/min | 15.0(0.8) | 67 (14) | 13.5(0.8) | 87 (6) | 16.4(1.6) | 93 (3) |
| 20 L/min | 10.2(0.5) | 66 (9) | *9.3(0.6)* | *89(4)* | *9.0 (0.6)* | *88 (10)* |
| 25 L/min | 9.3(0.6) | 77 (8) | *7.8(0.3)* | *91(5)* | *7.9(0.5)* | *93 (3)* |

**Table 3 - Chamber H**

| Volume → | 0.25 L | | 0.5 L | | 0.75 L | |
|---|---|---|---|---|---|---|
| Flow Rate | VMGD | ED | VMGD | ED | VMGD | ED |
| 15 L/min | 16.1 (0.8) | 57 (9) | 15.7 (0.7) | 78 (11) | 14.6 (1.1) | 90 (4) |
| 20 L/min | 12.0 (0.6) | 66 (9) | 10.4 (0.6) | 81 (7) | *10.2 (0.4)* | *89 (8)* |
| 25 L/min | 10.4 (0.6) | 75 (11) | *8.1 (0.3)* | *94 (4)* | *8.1 (0.3)* | *97 (1)* |

**Table 4 - Chamber I**

| Volume → | 0.25 L | | 0.5 L | | 0.75 L | |
|---|---|---|---|---|---|---|
| Flow Rate | VMGD | ED | VMGD | ED | VMGD | ED |
| 15 L/min | 18.2 (0.7) | 49 (8) | 19.3 (1.3) | 69 (12) | 18.2 (1.9) | 79 (12) |
| 20 L/min | 13.4 (0.5) | 43 (13) | 12.7 (1.0) | 71 (10) | 12.5 (0.6) | 83 (9) |
| 25 L/min | 12.0 (0.4) | 65 (S) | *10.0 (0.4)* | *85 (7)* | *9.7 (0.3)* | *87 (9)* |

In Tables 2-4, the italicized print indicates peak inspiratory flow rates and inhalation volumes at which the chambers achieved both the goal of an emitted dose of at least 85% and a dispersion of a VMGD of 11.8 µm or less. As is apparent from Tables 2-4, these goals were achieved for peak inspiratory flow rates of 25 L/min or less and for inhalation volumes of 0.75 L or less. Moreover, the standard deviations were quite small for the emitted dose (on the order of approximately 10% or less) and for the VMGD (on the order of approximately 1.0 or less).

In addition, statistical analysis was used to extrapolate the results from these three chambers into ranges of variables that would consistently yield the desired emitted dose and volume mean geometric diameter. For example, optimized combinations of chamber diameter, vent area, and mouthpiece hole area were determined. It should be apparent to one of ordinary skill in the art that optimization analysis could be performed for other variable combinations, and for other capsule sizes and powders, in order to optimize the design of the chambers.

Having done a thorough analysis, it has been determined that the present invention encompasses an optimized chamber, for a size 00 capsule, that has:
at least one aperture has an aggregate area of 0.116 to 0.142 cm² (0.018 to 0.022 square inches); or
a ring inner diameter of 0.965 to 1.016 cm (0. 380 to 0.400 inches); or
a chamber inner diameter of 1.016 to 1.118 cm (0.400 to 0.440 inches); or
three to five vents; or
a vent width of 0.051 to 0.064 cm (0.020 to 0.025 inches); or
a vent length of 0.495 to 0.599 cm (0.195 to 0.236 inches); or
a total vent area of 0.09 to 0.155 cm² (0.014 to 0.024 square inches),
and that when used with a dose of approximately 20 mg of the standard test powder described above and operated at a peak inspiratory flow rate of 25 L/min or less and an inhalation volume of 0.75 L or less, the emitted dose of powder will be at least 85%, and the VMGD will be about 11.8 µm or less.

While the preferred embodiment described above relates to optimizing the design of a chamber to have a have a resistance of at most 0.28 (cm H₂O)^{1/2}/L/min (0.088 (kPa)^{1/2}/L/min) and to provide an emitted dose of at least 85% when the dose of standard test powder is about 20 mg and when the device is operated at a peak inspiratory flow rate of 25 L/min or less and at an inhalation volume of 0.75 L or less, it should be understood that the invention also encompasses optimizing the chamber to have any other combination of resistance and emitted dose, at any other combination of powder type, dose weight, peak inspiratory flow rate, and inhalation volume.

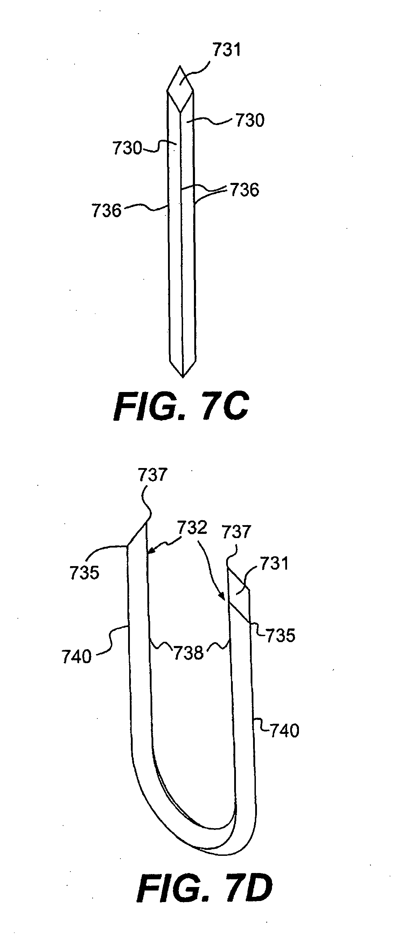

Turning now to FIGS. 7A through 7D, a preferred embodiment of the means for puncturing, in the form of a staple, suitable for use in the present invention is shown. The staple preferably comprises a rectangular length of material that has four planar side surfaces **730.** Each planar side surface intersects with two other planar side surfaces to create a total of four non-planar edges **736.** The staple is preferably bent into a substantially U-shaped configuration, thereby having a rounded portion and two prongs **732.** The prongs **732** terminate at two end surfaces **731.** As best seen in FIGS. 7A, 7C and 7D, end surfaces **731** are diamond-shaped.

The diamond-shaped end surfaces are created by bending the material about a non-planar edge. This configuration is best shown in FIGS. 7B and 7D. As can be seen, each prong **732** has an inner surface **738** that comprises one of the non-planar edges and an outer surface **740** that comprises the opposite non-planar edge. The inner surface **738** of each prong **732** terminates at the uppermost portion **737** of the diamond-shaped end surface, thereby creating a cutting edge for the prong. The outer surface **740** of the prong **732** terminates at the lowermost portion **735** of the diamond-shaped end surface.

FIGS. 9A and 9B depict another embodiment of a means for puncturing in the form of a staple, suitable for use in the present invention. This staple preferably comprises a rectangular length of material that has four planar side surfaces. Each planar side surface intersects with two other planar side surfaces to create a total of four non-planar edges. The staple is preferably bent into a substantially U-shaped configuration, thereby having a rounded portion and two prongs. The prongs terminate at two end surfaces that have a square shape.

The square-shaped end surfaces are created by bending the material about a planar side surface. As shown in FIG. 9A, each prong has an inner surface that comprises one of the planar side surfaces and an outer surface that comprises the opposite planar side surface. The inner surface of each prong terminates at the uppermost portion of the square-shaped end surface, thereby creating a cutting edge for the prong. The outer surface of the prong terminates at the lowermost portion of the square-shaped end surface.

FIG. 9B illustrates a puncture obtained from using the staple depicted in FIG. 9A. As shown, the holes formed by this staple have the appearance of being cut with a sharp edge. In addition, the material removed to create the hole is peeled back and remains well attached to the capsule; thereby preventing the capsule material from being inhaled by the user when the powder medicament is being dispensed.

FIG. 8 illustrates a puncture obtained from using the staple depicted in FIGS. 7A-7D. The holes formed by the staple appear to be cut with a sharp edge, and the excess material is peeled back. In testing, the effort required to puncture the capsule is lower than circular section staples, and approximately the same as a square section staple. However, during testing, no instances were noted of crushed or otherwise mispunctured capsules. These staples are extremely inexpensive to produce, approximately one-third the cost of square section staples such as those depicted in FIG 9A.

In addition to improved puncturing performance, drug delivery from capsules punctured with the staple depicted in FIGS. 7A-7D is greatly improved. The Emitted Dose (ED) and Fine Particle Fraction (FPF) of a test powder was measured at both 20 and 60 Liters per minute (LPM). In all cases, the aerosol emitted from capsules punctured with the diamond section staple of FIGS. 7A-7D was improved over a conventional circular stock staple. Most significantly, the FPF of powder delivered at 20 liters per minute was improved almost to the level of the FPF at 60 liters per minute.

FIGS. 16A through 16D illustrate yet another preferred embodiment of a means for puncturing suitable for use in the present invention, in the form of puncturing device **1600.** Puncturing device **1600** comprises two substantially longitudinal prongs **1620** coupled to a base **1610** coupled to form a U-shape. Base **1610** is configured to be coupled to inhalation device **100.** Although two prongs are illustrated in the figures, it should be understood that any number of prongs **1620** could be coupled to base **1610,** depending on the number of holes desired to be made in the receptacle. For ease of discussion, only one of prongs **1620** is described in detail below.

Prong **1620** has a proximal end coupled to the base **1610** and a distal end having a puncturing surface **1630** for making an initial puncture hole in the receptacle. In the embodiment shown, puncturing surface **1630** is a sharp point, although it should be understood that puncturing surface **1630** may also have a different shape, such as a sharp edge.

The periphery of prong **1620** further comprises a primary cutting edge **1640** running from the proximal end to the distal end of prong **1620** and terminating at puncturing surface **1630.** In a preferred embodiment, primary cutting edge **1640** is sharp and may have additional features to enhance its cutting ability, such as being serrated or jagged. The periphery also comprises substantially planar face **1650** running from the proximal end to the distal end of prong **1620.** In a preferred embodiment substantially planar face **1650** is substantially flat, although it may also be another suitable shape, such as slightly concave.

Prong **1620** further comprises a plurality of longitudinal edges **1645** and a plurality of longitudinal faces **1655** disposed around the periphery and running from the proximal end to the distal end of the prong. In a preferred embodiment, each of the longitudinal faces **1655** is substantially planar, although it should be understood that they may be other suitable shapes, such as concave. In a preferred embodiment, each of the longitudinal edges **1645** is sharp, although it should be understood that they may also have other suitable shapes, such as being serrated, jagged, blunt, or rounded.

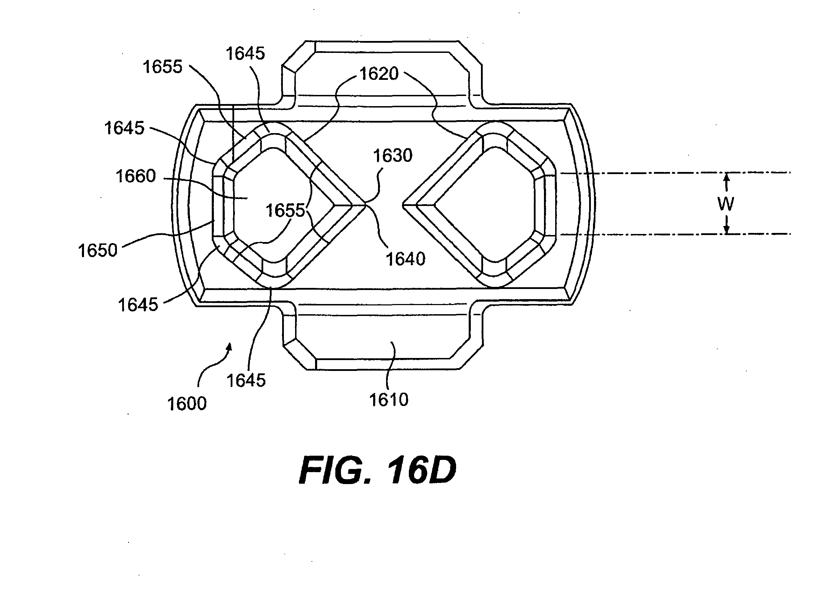

In the embodiment shown in FIG. 16D, there are four longitudinal edges **1645** and four longitudinal faces **1655,** in addition to primary cutting edge **1640** and substantially planar face **1650,** so that prong **1620** has a cross section substantially in the shape of a pentagon. However, it should be understood that there may be any number and arrangement of longitudinal faces **1655** and longitudinal edges **1645** so that prong **1620** may have other suitable cross sectional shapes, so long as substantially planar face **1650** is opposite to primary cutting edge **1640.** For example, width **W** (see FIG. 16D) of substantially planar face **1650** may be very small and the four longitudinal faces 1655 may be substantially at right angles to each other so that prong **1620** has substantially a diamond shaped cross section. In yet another embodiment, prong **1620** may have two longitudinal edges **1645** and two longitudinal faces **1655,** in addition to primary cutting edge **1640** and substantially planar face **1650,** so that prong **1620** has a triangular cross section.

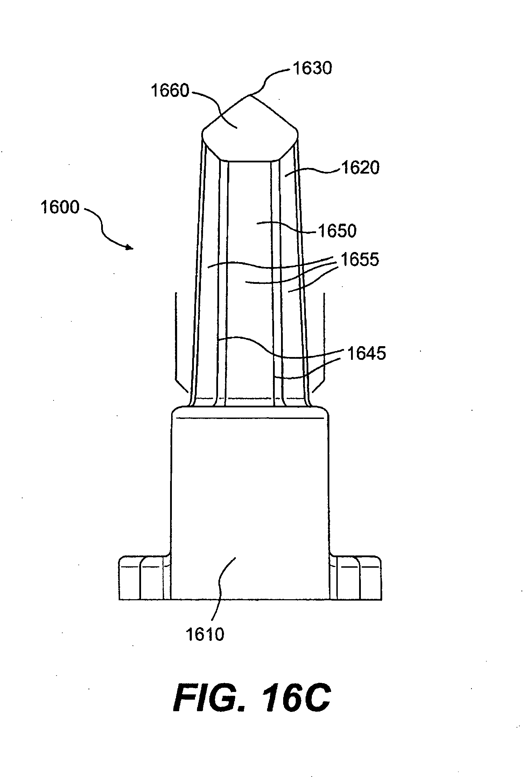

The distal end of prong **1620** preferably further comprises an angled face **1660** terminating in puncturing surface **1630** at its distal end and at substantially planar face **1650** at its proximal end, as best seen in FIG. 16C. It should be understood that angled face **1660** may be at any angle, or may be comprised of a plurality of angled faces at various angles, so long as puncturing surface **1630** is located distal to the distal end of substantially planar face **1650.**

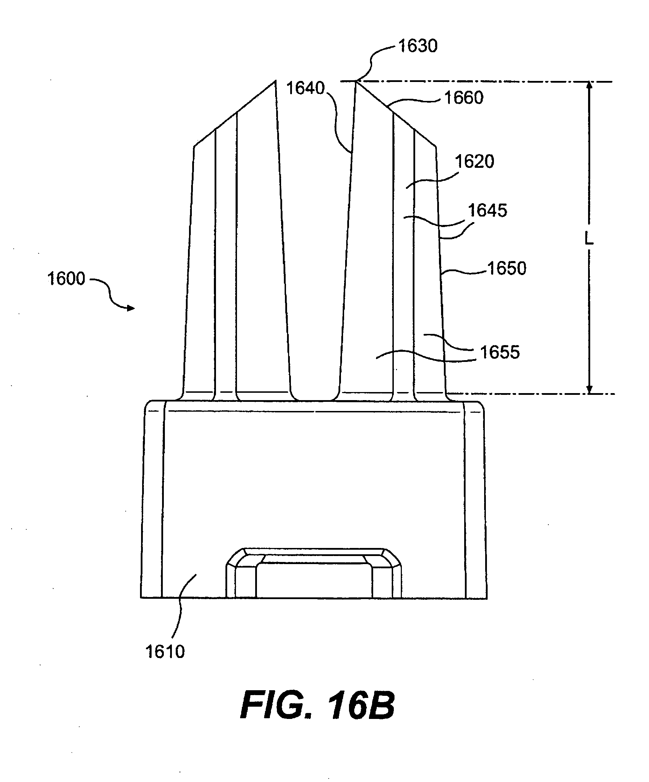

Also, as shown in FIG. 16B, prong **1620** is slightly tapered so that the distal end is smaller than the proximal end. This tapering facilitates removing prong **1620** from the wall to be punctured without sticking and without detaching the chad formed in the wall. In a preferred embodiment, the angle of the taper is approximately 0.116 degrees with respect to a longitudinal axis of the prong.

In a preferred embodiment, puncturing device **1600** is made by injection molding of a suitable metal, such as stainless steel or titanium. Injection molding facilitates making larger prongs than could be achieved in conventional piercing devices. As discussed above, larger prongs facilitate making larger holes in the receptacle in order to optimize the emitted dose and the volume mean geometric diameter. It should be understood that puncturing device **1600** may be made of another material, such as ceramic or plastic, or by another manufacturing process, such as casting or forging. Moreover, it should be understood that the other embodiments of means for puncturing **230** depicted in FIGS. 7A-D and 9A-B could be made by any of these manufacturing processes or materials. It should also be understood that these other embodiments of means for puncturing **230** could be coupled to a base similar to base **1610** in FIGS 16A-D.

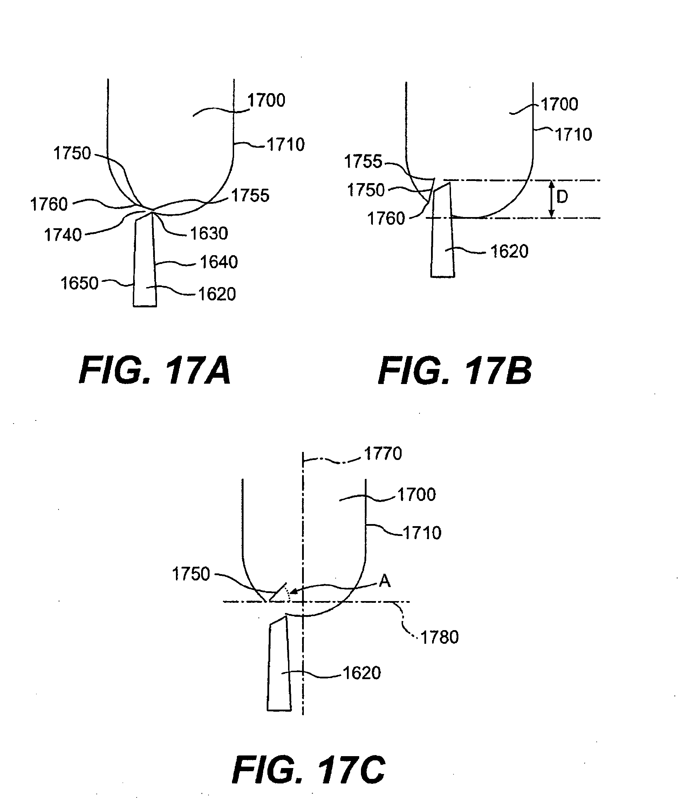

FIGS. 17A through 17D schematically illustrate the use of prong **1620** to puncture and create a hanging chad in the wall **1710** of receptacle **1700.** Although receptacle **1700** is illustrated in the shape of a capsule, it should be understood that the receptacle may have any other suitable shape, such as a tablet or a blister pack. Receptacle **1700** has a longitudinal axis **1770** substantially parallel to prong **1620** and a minor axis **1780** substantially perpendicular to longitudinal axis **1770.**

As shown in FIG. 17A, puncturing surface **1630** of prong **1620** initially punctures a small opening **1740** in wall **1710.** Next, as shown in FIG. 17B, prong **1620** is inserted into receptacle **1700** to a depth **D,** increasing the size of opening **1740** and forming chad **1750** having free end **1755.** Substantially planar face **1650** forms a hinge **1760** between chad **1750** and wall **1710** so that chad **1750** is a hanging chad. Finally, as shown in FIG. 17C, prong **1620** is withdrawn from wall **1710,** leaving handing chad **1750** inside of receptacle **1700.** Preferably, the angle **A** between chad **1750** and minor axis **1780,** after prong **1600** has been removed from receptacle **1700,** is at least 30 to 45 degrees in order to facilitate efficient emptying of the receptacle and a high emitted dose.

Several experiments were performed to evaluate the emitted doses achieved using puncturing device **1630.** The tests were done with size 00 capsules containing approximately 20 mg per capsule and using a flow rate of approximately 20 L/min for 1.5 seconds.

In the first experiment, two prototype staples similar in shape to the U-shaped staple shown in FIGS. 7A-7D but with larger prongs (referred herein as Staple #1 and Staple #2) were used to puncture ten capsules. For Staple #1, the mean emitted dose from the punctured capsules was approximately 81.0%, with a standard deviation of approximately 13.3%. For Staple #2, the mean emitted dose was approximately 51.0%, with a standard deviation of approximately 25.3%.

Next, the same experiments were run with Staple #1 and Staple #2, only this time the chads were manually opened to an angle of at least 45 degrees with respect to the receptacle after removal of the puncturing device, by using a blunt instrument. In that case, the mean emitted dose for Staple #1 was approximately 93.6%, with a standard deviation of approximately 2.4%. For Staple #2, the mean emitted dose was approximately 93.0% , with a standard deviation of approximately 2.0%.

The same experiments were then run using a prototype of the puncturing device 1600 illustrated in FIGS. 16A-D (called Staple #4). In the experiment performed without manually opening the chads to an angle of at least 45 degrees, the mean emitted dose after using Staple #4 was approximately 89.5%, with a standard deviation of approximately 4.9%. In the experiment in which the hanging chads were manually opened to an angle of at least **45** degrees, the mean emitted dose was approximately 93.9%, with a standard deviation of approximately 1.8%. By itself, Staple #4 opens the hanging chad to an angle of at least 30 to 45 degrees. Thus, the embodiment of puncturing device **1600** illustrated in FIGS. 16A-D has significant advantages over other puncturing means, including those previously described in this application, because it yields a consistent emitted dose of at least 85% and opens the chads to an angle of at least 30 to 45 degrees.

Other experiments were performed to determine the puncturing depth that could be achieved using puncturing device **1630.** First, Staple #3, another prototype having almost the same structure as Staples #1 and #2, was used to puncture capsules to varying depths. It was determined that the capsules could consistently be punctured to a depth of 0.1495 inches without causing chads to become removed. Next, Staple #5, another prototype of puncturing device **1600** illustrated in FIGS. 16A-D, was used to puncture capsules to varying depths. It was determined that the prongs could be inserted to a depth of at least 3/4 of the length L (see FIG. 16B) of the prongs, or approximately 0.2442 inches, without causing the chads to become removed. Accordingly, puncturing device **1600** illustrated in FIGS. 16A-D has significant advantages over other puncturing means because it allows greater depth of puncturing, which allows for greater optimization of the inhaler.

Also disclosed herein is a method for dispensing powder medicaments to a user through the various embodiments of the disclosed inhalation device. In such a method, a receptacle containing the powder medicament, e.g., a capsule **219,** is placed or formed into cylindrical chamber **210.** When the user compresses the inhalation device, staple **230** is moved toward capsule **219** thereby puncturing capsule **219** to cause the release of powder into chamber **210.** After release into the chamber, the powder is then inhaled by the user through apertures **224** and inhalation piece **226.** As noted, inhalation piece **226,** can be configured as either a mouth piece or a nose piece. For subsequent uses, the user merely replaces emptied capsule **219** with another capsule **219** that contains a new supply of power medicament. Alternatively, powder medicament is injected into a permanent receptacle that is formed into chamber **210.**

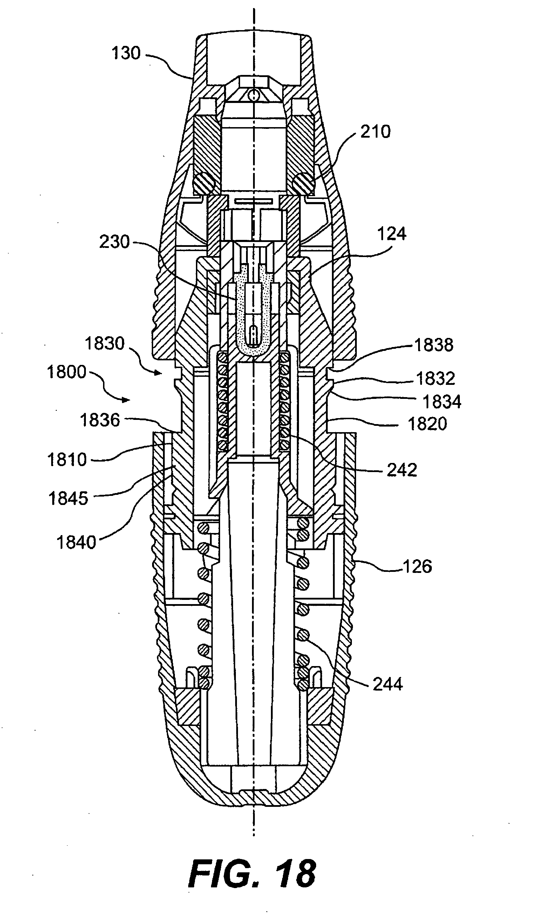

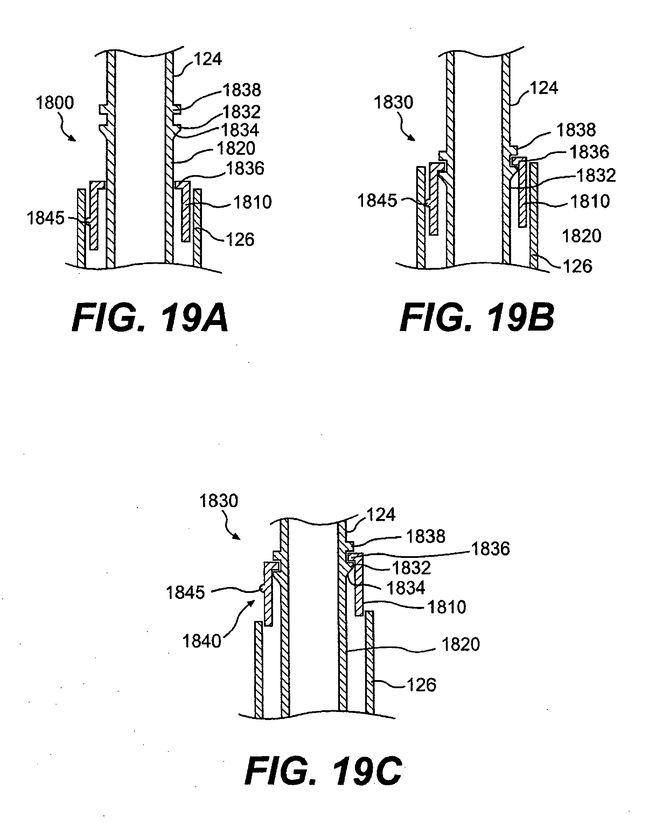

As shown in FIGS. 18 and 19A-19C, in another embodiment of the present invention, device **100** comprises a means for indicating readiness of the device for emitting powder **1800.** The means for indicating readiness **1800** comprises a body **1820** coupled to inner casing **124** and disposed in outer casing **126.** Body **1820** is reversibly moveable between a first position, as shown in FIGS. 18, 19A and 19C, and a second position, as shown in FIG. 19B. Body **1820** preferably is coupled to compression spring **244** so that it is biased in the first position. In a preferred embodiment, body **1820** comprises a hollow tube of oblong cross section, although it should be understood that body **1820** may have any other suitable shape, such as a round cylinder or rod.

Means for indicating readiness **1800** further comprises an indicator **1810** disposed in outer casing **126.** Indicator **1810** is reversibly moveable between a.rest position, as shown in FIGS. 18, 19A and 19B, and an indicating position, as shown in FIG. 19C. Indicator **1810** preferably comprises a hollow ring of oblong cross section, although it should be understood that indicator may have any other suitable shape, such as a round cylinder, a rod, or a plate.

Means for indicating readiness **1800** further comprises a means **1830** for coupling body **1820** and indicator **1810.** In a preferred embodiment, coupling means **1830** comprises at least one lip **1836** coupled to indicator **1810** and a corresponding at least one flange **1832** coupled to indicator **1810.** Each flange **1832** preferably comprises a ratchet surface **1834** to facilitate coupling and to prevent inadvertent decoupling of each lip **1836** and each flange **1832.** In addition, each flange **1832** preferable also comprises a stop **1838** to prevent indicator **1810** from riding up body **1820** beyond each flange **1832.** Although a preferred embodiment is illustrated, coupling means **1830** may comprise any other suitable structure for coupling body **1820** and indicator **1810,** such as, for example, a friction fit engagement, a plurality of corresponding tangs and grooves, a clip, or a hook and loop fastener.

In a preferred embodiment, as shown in FIG. 19A, before device **100** is actuated, body **1820** is biased in the first position and indicator **1810** is in the rest position and is substantially within outer casing **126,** so as to not be visible to the user. When device **100** is actuated to puncture a receptacle, body **1820** moves from the first position to the second position and further into outer casing **126,** as shown in FIG. 19B. When in the second position, coupling means **1830** causes body **1820** to become coupled to indicator **1810.** In the preferred embodiment illustrated in FIG. 19B, lip **1836** rides over ratchet surface **1834** of flange **1832** and becomes locked between flange **1832** and stop **1838.** Preferably, indicator **1810** makes an audible click when it becomes coupled to flange **1832,** which informs the user that the device has been actuated properly.

After device **100** is actuated, body **1820** is released and allowed to return to the first position, as shown in FIG. 19C. Because body **1820** is coupled to indicator **1810,** the movement of body **1820** to the first position causes indicator **1810** to move from the rest position to the indicating position. In the indicating position, indicator **1810** is at least partially outside of outer casing **126** so that indicator **1810** is visible to the user to indicate that device **100** is ready for inhalation. Indicator **1810** preferably has a bright color, such as, for example, green, to be easily visible.

Upon subsequent actuations of device **100,** indicator **1810** remains coupled to body **1820** and moves between the indicating position and the rest position as body **1820** moves between the first position and the second position, respectively, as shown in FIGS. 19B and 19C. In a preferred embodiment, indicator **1810** is equipped with a means **1840** for decoupling indicator **1810** from body **1820,** in order to return indicator **1810** to the rest position while body **1820** remains in the first position, as shown in FIG. 19A. The decoupling means **1840** is configured so that applying an axial force to indicator **1810** decouples indicator **1810** from body **1820.** In a preferred embodiment illustrated in the figures, decoupling means **1840** comprises at least one knob **1845** coupled to indicator **1810** to facilitate the user returning indicator **1810** to the rest position. It should be understood that decoupling means **1840** may have any other suitable structure, including a plurality of grooves or knobs or another type of easily graspable surface.

In the embodiment shown in FIGS. 19A-19C, indicator **1810** is disposed almost completely within outer casing **126** while in the rest position and is disposed partially within outer casing **126** when in the indicating position. However, it should be understood that a wide variety of other configurations are within the scope of the present invention. For example, indicator **1810** may be disposed substantially within outer casing **126** at both the rest position and the indicating position and may be viewable in one or both of these positions through a window in outer casing **126.** In another alternative embodiment, indicator **1810** may be disposed in upper casing portion **130.** In yet another alternative embodiment, indicator **1810** may be interchanged with body **1820** such that, for example, the body comprises a ring surrounding the indicator and the indicator is viewable through a window in the body and/or in the outer casing. In yet another alternative embodiment, indicator **1810** may be disposed in the indicating position before device **100** is ready for inhalation and in the rest position when device **100** is ready for inhalation, particularly in an embodiment in which indicator **1810** is viewable through a window in outer casing **126.**

Means for indicating **1800** may be used with any type of inhaler, or any another type of device that utilizes a body to which is applied an axial force. For example, in an alternative embodiment, means for indicating **1800** may be used to indicate that an epinephrine injection pen, used for treating allergies, has been used or is ready for use. In another alternative embodiment, means for indicating **1800** may be used to indicate that an aerosol canister inhaler has been used or is ready for use. Means for indicating **1800** may be used with both single-use and multiple-use devices. In addition, a device containing a plurality of inhalation chambers and a plurality of receptacles may comprise a plurality means for indicating **1800.**

### Conclusion

While various embodiments of the present invention have been described above, it should be understood that they have been presented by way of example only, and not limitation. For example, the present invention is not limited to the physical arrangements or dimensions illustrated or described. Nor is the present invention limited to any particular design or materials of construction. As such, the breadth and scope of the present invention should not be limited to any of the above-described exemplary embodiments, but should be defined only in accordance with the following claims and their equivalents.

## Claims

1. An inhalation device (100; 1500) for administering a dose of powder contained in a receptacle (219), comprising:
a chamber (210, 1510) configured to hold the receptacle (219), said chamber (210; 1510) defined by a wall (212; 1512) and having a proximal end (214), a distal end (216), and an inner surface, said wall (212; 1512) defining a plurality of vents (218; 1518), the chamber (210; 1510) optionally being cylindrical and/or having a ring (400) coupled to an inner surface of the chamber (210; 1510), and in that and
an inhalation portion (220; 1520) coupled to said proximal end (214) of said chamber (210;1510), said inhalation portion (220; 1520) defining at least one aperture (224;1524) configured to emit powder therethrough;
wherein the inhalation device (100; 1500) is configured to have a resistance of at most 0.088 (kPa)^{1/2}/L/min (0.28 (cmH₂O)^{1/2}/L/min) and to provide an emitted dose of at least 85% when the dose of powder is up to 20 mg and when the device (100; 1500) is operated at a peak inspiratory flow rate of 25 L/min or less and at an inhalation volume of 0.75 L or less.

2. The inhalation device (100; 1500) of claim 1, further comprising:
a first casing portion (120; 1540) and a second casing portion (130; 1550);
wherein the chamber (210; 1510) is a cylindrical chamber and comprises a ring (400) circumferentially coupled to the inner surface of the chamber (210; 1510), and the wall (212; 1512) is a straight wall of circular cross-section coupled to said first casing portion (120; 1540); and
wherein the second casing portion (130; 1550) is removably coupled to said first casing portion (120; 1540), said second casing portion (130; 1550) comprising the inhalation portion (220; 1520) which is disposed at the proximal end (214) of said chamber (210; 1510) when said first (120; 1540) and said second (130; 1550) casing portions are coupled, said inhalation portion (220; 1520) comprising a hemispheric region (222; 1522) defining a plurality of apertures (224; 1524) configured to emit powder therethrough.

3. The inhalation device (100; 1500) of claim 1 or claim 2, wherein a standard deviation of the emitted dose is 10% or less.

4. The inhalation device (100; 1500) of claim 1 or claim 2, wherein said at least one aperture (224; 1524) has an area of 0.116 cm² to 0.142 cm²(0.018 in² to 0.022 in²).

5. The inhalation device (100; 1500) of claim 1 or claim 2, wherein said ring (400) has an inner diameter of 0.965 cm to 1.016 cm (0.380 inches to 0.400 inches).

6. The inhalation device (100; 1500) of claim 1 or claim 2, wherein said cylindrical chamber (210; 1510) has an inner diameter of 1.016 cm to 1.118 cm (0. 400 inches to 0.440 inches).

7. The inhalation device (100; 1500) of claim 1 or claim 2, wherein a number of said plurality of vents (218; 1518) is three to five.

8. The inhalation device (100; 1500) of claim 1 or claim 2, wherein each of said plurality of vents (218; 1518) has a width of 0.051 cm to 0.064 cm (0.020 inches to 0.025 inches).

9. The inhalation device (100; 1500) of claim 1 or claim 2, wherein each of said plurality of vents (218; 1518) has a length of 0.495 cm to 0.599 cm (0.195 inches to 0.236 inches).

10. The inhalation device (100; 1500) of claim 1 or 2, wherein the plurality of vents (218; 1518) have an area of 0.09 cm² to 0.155 cm² (0.014 in² to 0.024 in²).

11. The inhalation device (100; 1500) of claim 1 or claim 2, wherein said at least one aperture (224; 1524) has an aggregate area of 0.129 cm² (0.020 in²), said ring (400) has an inner diameter of 1.016 cm (0.400 inches), said cylindrical chamber (210; 1510) has an inner diameter of 1.118 cm (0.440 inches), wherein a number of said plurality of vents (218; 1518) is three, each of said plurality of vents (218;1518) has a width of 0.051cm (0.020 inches), and each of said plurality of vents (218; 1518) has a length of 0.599 cm (0.236 inches).

12. The inhalation device (100; 1500) of claim 1 or claim 2, wherein said at least one aperture (224; 1524) has an aggregate area of 0.142 cm² (0.022 in²), said ring (400) has an inner diameter of 1.016 cm (0.400 inches), said cylindrical chamber (210; 1510) has an inner diameter of 1.118 cm (0.440 inches), wherein a number of said plurality of vents (218; 1518) is four, each of said plurality of vents (218; 1518) has a width of 0.051 cm (0.020 inches), and each of said plurality of vents (218; 1528) has a length of 0.599 cm (0.236 inches).

13. The inhalation device (100; 1500) of claim 1 or claim 2, wherein said at least one aperture (224; 1524) has an aggregate area of 0.142 cm² (0.022 in²), said ring (400) has an inner diameter of 0.965 cm (0.380 inches), said cylindrical chamber (210; 1510) has an inner diameter of 1.016 cm (0.400 inches), wherein a number of said plurality of vents (218; 1518) is five, each of said plurality of vents (218; 1518) has a width of 0.064 cm (0.025 inches), and each of said plurality of vents (218; 1518) has a length of 0.495 cm (0.195 inches).

14. The inhalation device (100; 1500) of claim 1 or claim 2, wherein the inhalation device (100; 1500) is configured to cause the powder to be highly dispersed.

15. The inhalation device (100; 1500) of claim 14, wherein the volume mean geometric diameter of the powder is 11.8 µm or less.

## Patentansprüche

1. Inhalationsgerät (100; 1500) zur Verabreichung einer Dosis eines in einem Behälter (219) enthaltenen Pulvers, mit
einer Kammer (210, 1510), die zum Halten des Behälters (219) ausgelegt ist, welche Kammer (210; 1510) von einer Wand (212; 1512) begrenzt ist und ein proximales Ende (214), ein distales Ende (216) und eine Innenfläche hat, wobei die Wand (212; 1512) eine Mehrzahl von Luftdurchlässen (218; 1518) definiert, die Kammer (210; 1510) gegebenenfalls zylindrisch ist und/oder einen Ring (400) hat, der mit einer Innenfläche der Kammer (210; 1510) gekuppelt ist, und mit
einem Inhalationsteil (220; 1520), der mit dem proximalen Ende (214) der Kammer (210; 1510) gekuppelt ist, welcher Inhalationsteil (220; 1520) mindestens eine Öffnung (224; 1524) festlegt, die ausgelegt ist, Pulver durch diese hindurch abzugeben;
wobei das Inhalationsgerät (100; 1500) so ausgelegt ist, dass es einen Widerstand von höchstens 0,088 (kPa)^{1/2}/l/min (0,28 (cmH₂O)^{1/2}/1/min) hat und eine abgegebene Dosis von mindestens 85% vorsieht, wenn die Pulver-Dosis bis zu 20 mg beträgt und wenn das Gerät (100; 1500) mit einem Spitzen-Ansaug-Durchsatz von 25 l/min oder weniger und mit einem Inhalationsvolumen von 0,75 1 oder weniger betrieben wird.

2. Inhalationsgerät (100; 1500) nach Anspruch 1, weiters mit:
einem ersten Gehäuseteil (120; 1540) und einem zweiten Gehäuseteil (130; 1550);
wobei die Kammer (210; 1510) eine zylindrische Kammer ist und einen Ring (400) aufweist, der am Umfang mit der Innenfläche der Kammer (210; 1510) gekuppelt ist, und die Wand (212; 1512) eine gerade Wand mit kreisförmigem Querschnitt ist, die mit dem ersten Gehäuseteil (120; 1540) gekuppelt ist; und
wobei der zweite Gehäuseteil (130; 1550) mit dem ersten Gehäuseteil (120; 1540) lösbar gekuppelt ist, der zweite Gehäuseteil (130; 1550) den Inhalationsteil (220; 1520) aufweist, der sich am proximalen Ende (214) der Kammer (210; 1510) befindet, wenn der erste (120; 1540) und der zweite (130; 1550) Gehäuseteil miteinander gekuppelt sind, wobei der Inhalationsteil (220; 1520) einen halbkugelförmigen Bereich (222; 1522) aufweist, der eine Mehrzahl von Öffnungen (224, 1524) definiert, die ausgelegt sind, Pulver durch diese hindurch abzugeben.

3. Inhalationsgerät (100; 1500) nach Anspruch 1 oder Anspruch 2, wobei eine Standardabweichung der abgegebenen Dosis 10% oder weniger ist.

4. Inhalationsgerät (100; 1500) nach Anspruch 1 oder Anspruch 2, wobei die mindestens eine Öffnung (224; 1524) eine Fläche von 0,116 cm² bis 0,142 cm² (0,018 Zoll² bis 0,022 Zoll²) hat.

5. Inhalationsgerät (100; 1500) nach Anspruch 1 oder Anspruch 2, wobei der Ring (400) einen Innendurchmesser von 0,965 cm bis 1,016 cm (0,380 Zoll bis 0,400 Zoll) hat.

6. Inhalationsgerät (100; 1500) nach Anspruch 1 oder Anspruch 2, wobei die zylindrische Kammer (210; 1510) einen Innendurchmesser von 1,016 cm bis 1,118 cm (0,400 Zoll bis 0,440 Zoll) hat.

7. Inhalationsgerät (100; 1500) nach Anspruch 1 oder Anspruch 2, wobei die Anzahl der Mehrzahl von Luftdurchlässen (218; 1518) drei bis fünf ist.

8. Inhalationsgerät (100; 1500) nach Anspruch 1 oder Anspruch 2, wobei jeder der Mehrzahl von Luftdurchlässen (218; 1518) eine Breite von 0,051 cm bis 0,064 cm (0,020 Zoll bis 0,025 Zoll) hat.

9. Inhalationsgerät (100; 1500) nach Anspruch 1 oder Anspruch 2, wobei jeder der Mehrzahl von Luftdurchlässen (218; 1518) eine Länge von 0,495 cm bis 0,599 cm (0,195 Zoll bis 0,236 Zoll) hat.

10. Inhalationsgerät (100; 1500) nach Anspruch 1 oder 2, wobei die Mehrzahl der Luftdurchlässe (218; 1518) eine Fläche von 0,09 cm² bis 0,155 cm² (0,014 Zoll² bis 0,024 Zoll²) hat.

11. Inhalationsgerät (100; 1500) nach Anspruch 1 oder Anspruch 2, wobei die mindestens eine Öffnung (224; 1524) eine Gesamtfläche von 0,129 cm² (0,020 Zoll²), der Ring (400) einen Innendurchmesser von 1,016 cm (0,400 Zoll) und die zylindrische Kammer (210; 1510) einen Innendurchmesser von 1,118 cm (0,440 Zoll) hat, wobei die Anzahl der Mehrzahl von Luftdurchlässen (218; 1518) drei ist, jeder der Mehrzahl von Luftdurchlässen (218; 1518) eine Breite von 0,051 cm (0,020 Zoll) und jeder der Mehrzahl von Luftdurchlässen (218; 1518) eine Länge von 0,599 cm (0,236 Zoll) hat.

12. Inhalationsgerät (100; 1500) nach Anspruch 1 oder Anspruch 2, wobei die mindestens eine Öffnung (224; 1524) eine Gesamtfläche von 0,142 cm² (0,022 Zoll²), der Ring (400) einen Innendurchmesser von 1,016 cm (0,400 Zoll) und die zylindrische Kammer (210; 1510) einen Innendurchmesser von 1,118 cm (0,440 Zoll) hat, wobei die Anzahl der Mehrzahl von Luftdurchlässen (218; 1518) vier ist, jeder der Mehrzahl von Luftdurchlässen (218; 1518) eine Breite von 0,051 cm (0,020 Zoll) und jeder der Mehrzahl von Luftdurchlässen (218; 1518) eine Länge von 0,599 cm (0,236 Zoll) hat.

13. Inhalationsgerät (100; 1500) nach Anspruch 1 oder Anspruch 2, wobei die mindestens eine Öffnung (224; 1524) eine Gesamtfläche von 0,142 cm² (0,022 Zoll²), der Ring (400) einen Innendurchmesser von 0,965 cm (0,380 Zoll), und die zylindrische Kammer (210; 1510) einen Innendurchmesser von 1,016 cm (0,400 Zoll) hat, wobei die Anzahl der Mehrzahl von Luftdurchlässen (218; 1518) fünf ist, jeder der Mehrzahl von Luftdurchlässen (218; 1518) eine Breite von 0,064 cm (0,025 Zoll) und jeder der Mehrzahl von Luftdurchlässen (218; 1518) eine Länge von 0,495 cm (0,195 Zoll) hat.

14. Inhalationsgerät (100; 1500) nach Anspruch 1 oder Anspruch 2, wobei das Inhalationsgerät (100; 1500) ausgelegt ist, eine starke Dispergierung des Pulvers zu bewirken.

15. Inhalationsgerät (100; 1500) nach Anspruch 14, wobei der volumenbezogene mittlere geometrische Durchmesser des Pulvers 11,8 µm oder weniger ist.

## Revendications

1. Dispositif d'inhalation (100 ; 1500) pour administrer une dose de poudre contenue dans un récipient (219), comprenant :
une chambre (210, 1510) configurée pour maintenir le récipient (219), ladite chambre (210 ; 1510) étant définie par une paroi (212 ; 1512) et ayant une extrémité proximale (214), une extrémité distale (216) et une surface interne, ladite paroi (212 ; 1512) définissant une pluralité d'évents (218 ; 1518), la chambre (210 ; 1510) étant éventuellement cylindrique et/ou ayant un anneau (400) couplé à une surface interne de la chambre (210 ; 1510), et
une partie d'inhalation (220 ; 1520) couplée à ladite extrémité proximale (214) de ladite chambre (210 ; 1510), ladite partie d'inhalation (220 ; 1520) définissant au moins une ouverture (224 ; 1524) configurée pour diffuser de la poudre au travers ;
dans lequel le dispositif d'inhalation (100 ; 1500) est configuré de manière à avoir une résistance maximale de 0,088 (kPa)^{1/2}/1/min (0,28 (cmH₂O)^{1/2}/1/min) et à fournir une dose diffusée d'au moins 85% lorsque la dose de poudre est présente jusqu'à 20 mg et lorsque le dispositif (100 ; 1500) fonctionne à un débit d'inspiration de pic de 25 1/min ou moins et à un volume d'inhalation de 0,75 1 ou moins.

2. Dispositif d'inhalation (100 ; 1500) selon la revendication 1, comprenant en outre :
une première partie de boîtier (120 ; 1540) et une seconde partie de boîtier (130 ; 1550) ;
dans lequel la chambre (210 ; 1510) est une chambre cylindrique et comprend un anneau (400) couplé sur la circonférence à la surface interne de la chambre (210; 1510) et la paroi (212 ; 1512) est une paroi droite de section transversale circulaire couplée à ladite première partie de boîtier (120 ; 1540) ; et
dans lequel la seconde partie de boîtier (130 ; 1550) est couplée de manière amovible à ladite première partie de boîtier (120 ; 1540), ladite seconde partie de boîtier (130 ; 1550) comprenant la partie d'inhalation (220 ; 1520) qui est disposée à l'extrémité proximale (214) de ladite chambre (210 ; 1510) lorsque lesdites première (120 ; 1540) et seconde (130 ; 1550) parties de boîtier sont couplées, ladite partie d'inhalation (220 ; 1520) comprenant une région hémisphérique (222 ; 1522) définissant une pluralité d'ouvertures (224 ; 1524) configurées pour diffuser de la poudre au travers.

3. Dispositif d'inhalation (100 ; 1500) selon les revendications 1 ou 2, dans lequel un écart type de la dose diffusée est de 10% ou moins.

4. Dispositif d'inhalation (100 ; 1500) selon la revendication 1 ou la revendication 2, dans lequel ladite au moins une ouverture (224 ; 1524) a une superficie de 0,116 cm² à 0,142 cm² (0,018 pouce² à 0,022 pouce²).

5. Dispositif d'inhalation (100 ; 1500) selon la revendication 1 ou la revendication 2, dans lequel ledit anneau (400) a un diamètre interne de 0,965 cm à 1,016 cm (0,380 pouce à 0,400 pouce).

6. Dispositif d'inhalation (100 ; 1500) selon la revendication 1 ou la revendication 2, dans lequel ladite chambre cylindrique (210 ; 1510) a un diamètre interne de 1,016 cm à 1,118 cm (0,400 pouce à 0,440 pouce).

7. Dispositif d'inhalation (100 ; 1500) selon la revendication 1 ou la revendication 2, dans lequel le nombre de ladite pluralité d'évents (218 ; 1518) est de trois à cinq.

8. Dispositif d'inhalation (100 ; 1500) selon la revendication 1 ou la revendication 2, dans lequel chaque évent de ladite pluralité d'évents (218 ; 1518) présente une largeur de 0,051 cm à 0,064 cm (0,020 pouce à 0,025 pouce).

9. Dispositif d'inhalation (100 ; 1500) selon la revendication 1 ou la revendication 2, dans lequel chaque évent de ladite pluralité d'évents (218 ; 1518) présente une longueur de 0,495 cm à 0,599 cm (0,195 pouce à 0,236 pouce).

10. Dispositif d'inhalation (100 ; 1500) selon la revendication 1 ou la revendication 2, dans lequel la pluralité d'évents (218 ; 1518) a une superficie de 0,09 cm² à 0,155 cm² (0,014 pouce² à 0,024 pouce²).

11. Dispositif d'inhalation (100 ; 1500) selon la revendication 1 ou la revendication 2, dans lequel ladite au moins une ouverture (224 ; 1524) a une superficie globale de 0,129 cm² (0,020 pouce²), ledit anneau (400) a un diamètre interne de 1,016 cm (0,400 pouce), ladite chambre cylindrique (210 ; 1510) a un diamètre interne de 1,118 cm (0,440 pouce), dans lequel le nombre de ladite pluralité d'évents (218 ; 1518) est de trois, chacun de ladite pluralité d'évents (218 ; 1518) a une largeur de 0,051 cm (0,020 pouce) et chacun de ladite pluralité d'évents (218 ; 1518) a une longueur de 0,599 cm (0,236 pouce).

12. Dispositif d'inhalation (100 ; 1500) selon la revendication 1 ou la revendication 2, dans lequel ladite au moins une ouverture (224 ; 1524) a une superficie globale de 0,142 cm² (0,022 pouce²), ledit anneau (400) a un diamètre interne de 1,016 cm (0,400 pouce), ladite chambre cylindrique (210 ; 1510) a un diamètre interne de 1,118 cm (0,440 pouce), dans lequel le nombre de ladite pluralité d'évents (218 ; 1518) est de quatre, chacun de ladite pluralité d'évents (218 ; 1518) a une largeur de 0,051 cm (0,020 pouce) et chacun de ladite pluralité d'évents (218 ; 1518) a une longueur de 0,599 cm (0,236 pouce).

13. Dispositif d'inhalation (100 ; 1500) selon la revendication 1 ou la revendication 2, dans lequel ladite au moins une ouverture (224 ; 1524) a une superficie globale de 0,142 cm² (0,022 pouce²), ledit anneau (400) a un diamètre interne de 0,965 cm (0,380 pouce), ladite chambre cylindrique (210 ; 1510) a un diamètre interne de 1,016 cm (0,400 pouce), dans lequel le nombre de ladite pluralité d'évents (218 ; 1518) est de cinq, chacun de ladite pluralité d'évents (218 ; 1518) a une largeur de 0,064 cm (0,025 pouce) et chacun de ladite pluralité d'évents (218 ; 1518) a une longueur de 0,495 cm (0,195 pouce).

14. Dispositif d'inhalation (100 ; 1500) selon la revendication 1 ou la revendication 2, dans lequel le dispositif d'inhalation (100 ; 1500) est configuré pour assurer une haute dispersion de la poudre.

15. Dispositif d'inhalation (100 ; 1500) selon la revendication 14, dans lequel le diamètre géométrique moyen en volume de la poudre est de 11,8 µm ou moins.
